(19) **Europäisches Patentamt European Patent Office Office européen des brevets**

(11) **EP 4 484 627 A1**

(12) **EUROPEAN PATENT APPLICATION**
published in accordance with Art. 153(4) EPC

(43) Date of publication:
**01.01.2025 Bulletin 2025/01**

(21) Application number: **23780083.4**

(22) Date of filing: **23.03.2023**

(51) International Patent Classification (IPC):
**D04H 3/16** (2006.01)        **D01F 6/46** (2006.01)
**D01F 8/06** (2006.01)        **D01F 8/16** (2006.01)
**D04H 3/007** (2012.01)       **D04H 3/009** (2012.01)
**D04H 3/147** (2012.01)

(52) Cooperative Patent Classification (CPC):
**A61F 13/511; D01F 6/46; D01F 8/06; D01F 8/16; D04H 3/007; D04H 3/009; D04H 3/147; D04H 3/16**

(86) International application number:
**PCT/JP2023/011623**

(87) International publication number:
**WO 2023/190073 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.03.2022 JP 2022057478**

(71) Applicant: **Mitsui Chemicals Asahi Life Materials Co., Ltd.**
**Tokyo 104-0028 (JP)**

(72) Inventors:
• **INOUE, Fumihito**
  **Sodegaura-shi, Chiba 299-0265 (JP)**
• **IIHAMA, Sho**
  **Sodegaura-shi, Chiba 299-0265 (JP)**
• **IIBA, Kouzou**
  **Tokyo 104-0028 (JP)**

(74) Representative: **Hoffmann Eitle**
**Patent- und Rechtsanwälte PartmbB**
**Arabellastraße 30**
**81925 München (DE)**

(54) **SPUN-BOND NON-WOVEN FABRIC, HYGIENIC MATERIAL, AND METHOD FOR PRODUCING SPUN-BOND NON-WOVEN FABRIC**

(57)    The spun-bonded nonwoven fabric of the present disclosure contains fibers formed of a resin composition. The resin composition contains a propylene polymer (A), a thermoplastic polyurethane-based elastomer (B), and a polymer (C) modified with at least one polar group selected from oxygen-containing groups, nitrogen-containing groups, sulfur-containing groups, or phosphorus-containing groups. A ratio of a peak intensity of $\alpha$-crystals of the fibers with respect to a crystallinity of the fibers is 10.5 or lower. The peak intensity of the $\alpha$-crystals represents a peak intensity of a (110) plane of the propylene polymer (A) at approximately $2\theta = 14°$ in an X-ray diffraction pattern measured by an X-ray diffractometer using CuK$\alpha$ radiation.

EP 4 484 627 A1

## Description

Technical Field

The present disclosure relates to: a spun-bonded nonwoven fabric; a hygienic material; and a method of producing a spun-bonded nonwoven fabric.

Background Art

In recent years, nonwoven fabrics formed of fibers containing a thermoplastic resin are widely used in various applications because of their excellent air permeability, flexibility, lightweightness, etc. As methods of producing a nonwoven fabric, melt-blowing methods and spunbonding methods that are suitable for mass production have been widely used.

Particularly, nonwoven fabrics produced by a spunbonding method (hereinafter, also referred to as "spun-bonded nonwoven fabrics") are used in many fields since they have excellent continuous spinnability and productivity, in addition to being excellent in mechanical properties such as tensile strength, as well as properties such as bending resistance and air permeability.

Examples of thermoplastic resins used for such spun-bonded nonwoven fabrics, etc. include polyamide resins, polyester resins, and polyolefin resins from the viewpoint of melt spinnability, fiber characteristics, etc. Especially, in absorbent articles, polyolefin resins that are inexpensive and excellent in workability are often used.

Patent Document 1 discloses a spun-bonded nonwoven fabric formed from fibers containing a propylene polymer. In the spun-bonded nonwoven fabric disclosed in Patent Document 1, the MFR (melt flow rate), the fineness, the basis weight, and the embossed area ratio are each in a prescribed range.

Patent Document 1: WO 2007/091444

SUMMARY OF THE INVENTION

Technical Problem

The spun-bonded nonwoven fabric disclosed in Patent Document 1 had room for improvement in terms of satisfying both tensile strength and air permeability.

Embodiments of the present disclosure were made, and an object of the disclosure is to provide: a spun-bonded nonwoven fabric having an excellent tensile strength and a high air permeability; a hygienic material; and a method of producing a spun-bonded nonwoven fabric.

Solution to Problem

Means for solving the above-described problems encompass the following embodiments.

<1> A spun-bonded nonwoven fabric, containing fibers formed of a resin composition that contains:

a propylene polymer (A);
a thermoplastic polyurethane-based elastomer (B); and
a polymer (C) modified with at least one polar group selected from oxygen-containing groups, nitrogen-containing groups, sulfur-containing groups, or phosphorus-containing groups,
wherein:

a ratio of a peak intensity of an $\alpha$-crystals of the fibers with respect to a crystallinity of the fibers is 10.5 (intensity(counts)/%) or lower, and
the peak intensity of the $\alpha$-crystals represents a peak intensity of a (110) plane of the propylene polymer (A) at approximately $2\theta = 14°$ in an X-ray diffraction pattern measured by an X-ray diffractometer using CuK$\alpha$ radiation.

<2> A spun-bonded nonwoven fabric, containing fibers formed of a resin composition that contains:

a propylene polymer (A);
a crystal nucleating agent (b); and
a polymer (C) modified with at least one polar group selected from oxygen-containing groups, nitrogen-containing groups, sulfur-containing groups, or phosphorus-containing groups,
wherein:

the fibers have a sea-island structure,
a sea phase contained in the sea-island structure contains the propylene polymer (A),
island phases contained in the sea-island structure contain the crystal nucleating agent (b),
a ratio of a peak intensity of an $\alpha$-crystals of the fibers with respect to a crystallinity of the fibers is 10.5 (intensity(counts)/%) or lower, and
the peak intensity of the $\alpha$-crystals represents a peak intensity of a (110) plane of the propylene polymer (A) at approximately $2\theta = 14°$ in an X-ray diffraction pattern measured by an X-ray diffractometer using CuK$\alpha$ radiation.

<3> The spun-bonded nonwoven fabric according to <1>, wherein:

a content of the propylene polymer (A) is from 70.0% by mass to 97.0% by mass with respect to a total amount of the resin composition,
a content of the thermoplastic polyurethane-based elastomer (B) is from 2.0% by mass to 20.0% by mass with respect to the total amount of the resin composition, and
a content of the polymer (C) is from 1.0% by mass to 10.0% by mass with respect to the total amount of the resin composition.

<4> The spun-bonded nonwoven fabric according to <1> or <3>, wherein, in a cross-section of a fiber perpendicular to the axial direction of the fiber observed under a transmission electron microscope in a viewing area of 25.5 $\mu m^2$;

island phases containing the thermoplastic polyurethane-based elastomer (B) have an average diameter of 0.095 $\mu m$ or less; and
a number of the island phases containing the thermoplastic polyurethane-based elastomer (B) is 200 or more.

<5> The spun-bonded nonwoven fabric according to any one of <1> to <4>, wherein the polar group contains at least one of oxygen-containing groups or nitrogen-containing groups.
<6> The spun-bonded nonwoven fabric according to any one of <1> to <5>, wherein the polar group contains at least one of amino groups, carboxyl groups, carboxylic acid anhydride groups, or ester groups.
<7> The spun-bonded nonwoven fabric according to <6>, wherein the polar group contains amino groups.
<8> The spun-bonded nonwoven fabric according to any one of <1> to <7>, wherein a main chain of the polymer (C) contains a polyolefin or a hydrogenated styrene-based block copolymer.
<9> The spun-bonded nonwoven fabric according to any one of <1> to <8>, wherein the propylene polymer (A) contains a propylene homopolymer having a melting point of 140°C or higher.
<10> The spun-bonded nonwoven fabric according to any one of <1> to <9>, wherein the resin composition further contains a polyethylene (D) having a density of from 0.941 g/cm$^3$ to 0.970 g/cm$^3$.
<11> The spun-bonded nonwoven fabric according to <10>, wherein a content of the polyethylene (D) is from 1.0% by mass to 10.0% by mass with respect to a total amount of the resin composition.
<12> The spun-bonded nonwoven fabric according to <10> or <11>, wherein, in a cross-section of a fiber perpendicular to the axial direction of the fiber observed under a transmission electron microscope in a viewing area of 25.5 $\mu m^2$;

island phases containing the polyethylene (D) have an average diameter of 0.11 $\mu m$ or less; and
a number of the island phases containing the polyethylene (D) is 150 or more.

<13> The spun-bonded nonwoven fabric according to any one of <1> to <12>, wherein the ratio of a peak intensity of an $\alpha$-crystals of the fibers with respect to a crystallinity of the fibers is from 8.5 (intensity(counts)/%) to 10.5 (intensity(counts)/%).
<14> A hygienic material, including the spun-bonded nonwoven fabric according to any one of <1> to <13>.
<15> A method of producing a spun-bonded nonwoven fabric, the method including:

melt-spinning a resin composition to form a continuous fiber group; and
stretching the continuous fiber group with a cooling air,
wherein:

the resin composition contains a propylene polymer (A), a thermoplastic polyurethane-based elastomer (B), and a polymer (C) modified with at least one polar group selected from oxygen-containing groups, nitrogen-containing groups, sulfur-containing groups, or phosphorus-containing groups, and
the cooling air has a flow rate of 3,000 m/min or more.

Advantageous Effects of Invention

According to embodiments of the disclosure, the following are provided: a spun-bonded nonwoven fabric having an excellent tensile strength and a high air permeability; a hygienic material; and a method of producing a spun-bonded nonwoven fabric.

BRIEF DESCRIPTION OF DRAWING

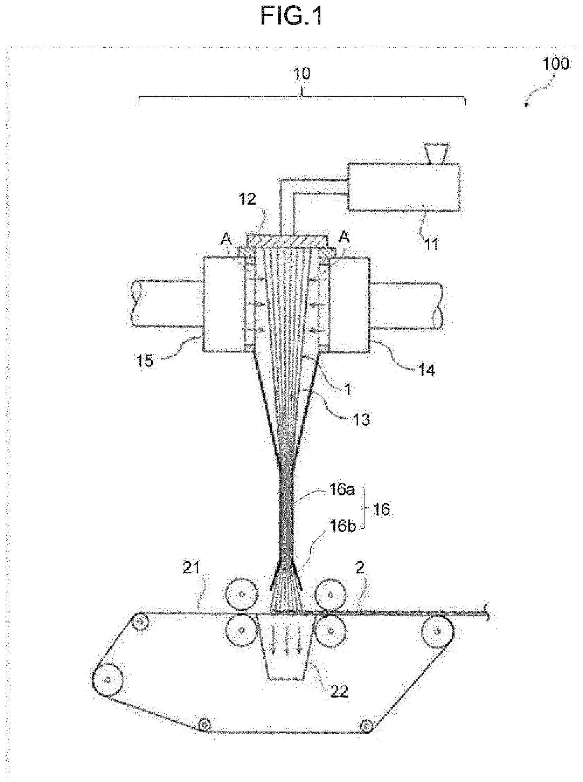

FIG. 1 is a schematic drawing that illustrates one example of a production apparatus used for a closed-system spunbonding method.

DESCRIPTION OF EMBODIMENTS

Embodiments of the disclosure will now be described. The following descriptions and Examples exemplify the embodiments, and do not limit the scope of the embodiments.
In the disclosure, a combination of two or more preferred aspects is a more preferred aspect.
In the disclosure, those numerical ranges that are expressed with "to" each denote a range that includes the numerical values stated before and after "to" as the lower limit value and the upper limit value, respectively.
In the disclosure, the term "step" encompasses not only a discrete step but also a step that cannot be clearly distinguished from other steps, as long as the intended purpose of the step is achieved.
In a set of numerical ranges that are stated in a stepwise manner in the disclosure, the upper limit value or the lower limit value of one numerical range may be replaced with the upper limit value or the lower limit value of other numerical range. Further, in a numerical range stated in the disclosure, the upper limit value or the lower limit value of the numerical range may be replaced with a relevant value indicated in any of Examples.
In the disclosure, each component may contain plural substances that correspond to the component. In the disclosure, where the amount of a component in a composition is mentioned and there are plural substances that correspond to the component of the composition, the indicated amount of the component in the composition means, unless otherwise specified, a total amount of the plural substances existing in the composition.
In the disclosure, "% by mass" and "% by weight" are synonymous, and "parts by mass" and "parts by weight" are synonymous. In the disclosure, unless otherwise specified, "%" indicating the amount of a contained component is based on mass.
The term "MD (Machine Direction)" used herein refers to a moving direction of a nonwoven fabric web in a nonwoven fabric production apparatus. The term "CD (Cross Direction)" used herein refers to a direction that is perpendicular to the MD and parallel to a main surface (a surface perpendicular to the thickness direction of a nonwoven fabric).
The term "layer" used herein encompasses, when a region having the layer is observed, not only a case where the layer is formed on the entirety of the region but also a case where the layer is formed only a part of the region.
In the disclosure, where a group (atomic group) is described without an indication of whether it is substituted or unsubstituted, the group encompasses both cases of having a substituent and not having a substituent.
The term "compatibility used herein refers to a state in which two different kinds of substances (polymers in particular) are uniformly mixed. The two different kinds of substances may be completely or partially compatible with each other.

(1) First Aspect

(1.1) Spun-Bonded Nonwoven Fabric

The spun-bonded nonwoven fabric according to a first aspect contains fibers formed of a resin composition. This resin composition contains: a propylene polymer (A); a thermoplastic polyurethane-based elastomer (B); and a polymer (C) modified with at least one polar group selected from oxygen-containing groups, nitrogen-containing groups, sulfur-containing groups, or phosphorus-containing groups (hereinafter, simply referred to as "polymer (C)"). A ratio of a peak intensity of $\alpha$-crystals of the fibers with respect to a crystallinity of the fibers (hereinafter, also referred to as "ratio (peak intensity/crystallinity)") is 10.5 (intensity(counts)/%) or lower. The peak intensity of the $\alpha$-crystals represents a peak intensity of a (110) plane of the propylene polymer (A) at approximately $2\theta = 14°$ in an X-ray diffraction pattern measured by an X-ray diffractometer using CuK$\alpha$ radiation.

The term "spun-bonded nonwoven fabric" used herein refers to a nonwoven fabric produced by one or more bonding methods in which a continuous fiber group generated by spinning a molten or dissolved resin composition from a spinneret is disposed in layers on a moving collection member (e.g., a net conveyer). A continuous fiber is also referred to as "filament".

The term "polymer (C) modified with a polar group" refers to a polymer which includes a main chain that contains a hydrocarbon having a relatively low polarity, and a polar group that is bound to the main chain and has a relatively high polarity.

The term "polar group" used herein refers to a group containing at least one heteroatom. Examples of the heteroatom include an oxygen atom, a nitrogen atom, a sulfur atom, a silicon atom, a phosphorus atom, a chlorine atom, an iodine atom, and a bromine atom.

The term "crystallinity of fibers" refers to a ratio in percentage (%) of a peak area of crystalline parts with respect to a total of the peak area of crystalline parts and an area of amorphous parts in an X-ray diffraction pattern measured by an X-ray diffractometer using CuK$\alpha$ radiation.

The spun-bonded nonwoven fabric according to the first aspect has the above-described configuration; therefore, it exhibits an excellent tensile strength and a high air permeability. In other words, in the spun-bonded nonwoven fabric according to the first aspect, an effect of improving the air permeability while maintaining or improving the tensile strength is exerted. The tensile strength and the air permeability are in a general trade-off relationship.

This effect is presumably attributed to, but not limited to, the following reasons.

The thermoplastic polyurethane-based elastomer (B) has a higher density than the propylene polymer (A) and can thus improve the fiber strength of the spun-bonded nonwoven fabric. As a result, in the spun-bonded nonwoven fabric, the strength itself can be maintained even when the amount of the fibers per unit volume is reduced.

However, the thermoplastic polyurethane-based elastomer (B) and the propylene polymer (A) may be incompatible with each other in a non-compatible resin composition obtained by simply adding the thermoplastic polyurethane-based elastomer (B) to the propylene polymer (A). This is presumably because the propylene polymer (A) is a resin having a relatively low polarity while the thermoplastic polyurethane-based elastomer (B) is a resin having a relatively high polarity, and their compatibility is thus poor. Accordingly, in crystal structures of fibers formed of such a non-compatible resin composition, the thermoplastic polyurethane-based elastomer (B) forms relatively large island phases. Thus, in an attempt to produce fibers from the non-compatible resin composition using a production apparatus for a spunbonding method (hereinafter, also simply referred to as "apparatus"), a load is locally applied to the inside of the resulting fibers, and the non-compatible resin composition may not be able to be fierized.

In view of the above, in the first aspect, the resin composition contains the polymer (C) in addition to the propylene polymer (A) and the thermoplastic polyurethane-based elastomer (B). Thereby, the compatibility of the propylene polymer (A) and the thermoplastic polyurethane-based elastomer (B) is improved. This is presumably because the polymer (C) functions as a compatibilizer since the main chain of the polymer (C) has a high affinity for the propylene polymer (A), and the polar group of the polymer (C) has a high affinity for the thermoplastic polyurethane-based elastomer (B). This allows island phases of the thermoplastic polyurethane-based elastomer (B) to be microdispersed in the crystal structures of the fibers formed of the resin composition according to the first aspect (i.e., the average diameter of the island phases are further reduced, and a number of the island phases is thus increased). In other words, fibers in which crystal structures are more uniform (i.e., fibers having a more uniform structure) can be obtained. Accordingly, the fibers are more uniformly dispersed by the apparatus and deposited on a moving collection surface of the apparatus. As a result, it is presumed that the resulting spun-bonded nonwoven fabric has a high air permeability.

Further, in the first aspect, the ratio (peak intensity/crystallinity) is 10.5 (intensity(counts)/%) or lower. The feature that the ratio (peak intensity/crystallinity) is 10.5 (intensity(counts)/%) or lower indicates not only that the $\alpha$-crystals amount of the propylene polymer (A) is relatively smaller than the amount of fibers formed of a non-compatible resin composition, but also that a hard crystal phase and a soft amorphous phase do not have a clear boundary therebetween, and that the ordered structure change of the crystals of the propylene polymer (A) and the thermoplastic polyurethane-based elastomer (B) is continuous, allowing the fibers themselves to have a relatively uniform crystal structure. Thereby, the fibers have a relatively high strength. As a result, the resulting spun-bonded nonwoven fabric is presumed to have an excellent tensile strength.

From the above, the spun-bonded nonwoven fabric according to the first aspect is presumed to have an excellent tensile strength and a high air permeability.

(1.1.1) Fibers

The spun-bonded nonwoven fabric according to the first aspect contains fibers.

(1.1.1.1) Ratio (Peak intensity/Crystallinity)

The ratio (peak intensity/crystallinity) is 10.5 (intensity(counts)/%) or lower. The peak intensity of the $\alpha$-crystals represents a peak intensity of the (110) plane of the propylene polymer (A) at approximately 20 = 14° in an X-ray diffraction pattern measured by an X-ray diffractometer using CuK$\alpha$ radiation.

Methods of measuring each of the crystallinity of the fibers and the peak intensity of the $\alpha$-crystals of the fibers are the same as the methods described below in the section of Examples.

The ratio (peak intensity/crystallinity) indicates the amount of $\alpha$-crystals per unit crystal. A low ratio (peak intensity/crystallinity) means that the amount of complete $\alpha$-crystals per unit crystal is small. In other words, it is considered that the boundary between a crystal phase (and amorphous phase) of the propylene polymer (A) and a crystal phase (and amorphous phase) of the thermoplastic polyurethane-based elastomer (B) is small, and these crystal phases form a uniform crystal system.

The ratio (peak intensity/crystallinity) is 10.5 (intensity(counts)/%) or lower. This allows the spun-bonded nonwoven fabric to have a superior tensile strength and a higher air permeability.

From the viewpoint of obtaining both satisfactory tensile strength and satisfactory air permeability in the spun-bonded nonwoven fabric, the ratio (peak intensity/crystallinity) is preferably from 8.5 (intensity(counts)/%) to 10.5 (intensity(counts)/%).

From the viewpoint of obtaining both satisfactory tensile strength and satisfactory air permeability in the spun-bonded nonwoven fabric, the ratio (peak intensity/crystallinity) is more preferably 10.3 (intensity(counts)/%) or lower, still more preferably 10.0 (intensity(counts)/%) or lower, particularly preferably 9.7 (intensity(counts)/%) or lower.

From the viewpoint of obtaining both satisfactory tensile strength and satisfactory air permeability in the spun-bonded nonwoven fabric, the ratio (peak intensity/crystallinity) is more preferably 8.8 (intensity(counts)/%) or higher, still more preferably 9.0 (intensity(counts)/%) or higher, particularly preferably 9.2 (intensity(counts)/%) or higher.

As a method of adjusting the ratio (peak intensity/crystallinity) to be 10.5 (intensity(counts)/%) or lower, for example, the below-described method of producing a spun-bonded nonwoven fabric may be employed.

A peak intensity of the $\alpha$-crystals of the fibers is not particularly limited and, from the viewpoint of improving the tensile strength of the spun-bonded nonwoven fabric, it is preferably from 500 (intensity(counts)) to 570 (intensity(counts)), more preferably from 510 (intensity(counts)) to 560 (intensity(counts)), still more preferably from 520 (intensity(counts)) to 550 (intensity(counts)).

As a method of adjusting the peak intensity of the $\alpha$-crystals of the fibers to be in the above-described range, for example, the below-described method of producing a spun-bonded nonwoven fabric may be employed.

A crystallinity of the fibers is not particularly limited and, from the viewpoint of improving the tensile strength of the spun-bonded nonwoven fabric, it is preferably from 50% to 60%, more preferably from 51% to 58%, still more preferably from 52% to 56%.

As a method of adjusting the crystallinity of the fibers to be in the above-described range, for example, the below-described method of producing a spun-bonded nonwoven fabric may be employed.


(1.1.1.2) Sea-Island Structure

In the first aspect, the fibers may have a sea-island structure.

The expression "fibers have a sea-island structure" used herein means that cross-sections of the fibers cut in a direction perpendicular to the axial direction of the fibers have a sea-island structure.

The term "sea-island structure" used herein refers to a phase-separated structure in which phases (i.e., island phases) containing one of at least two components exist (e.g., are dispersed) in a continuous phase (i.e., sea phase) formed of the other component.

Hereinafter, "fibers having a sea-island structure" are also referred to as "sea-island fibers".

In the first aspect, the sea-island fibers are formed of a resin composition. This resin composition contains a propylene polymer (A), a thermoplastic polyurethane-based elastomer (B), and a polymer (C), and may further contain a polyethylene (D) if necessary. The details of the resin composition will be described below.

The sea-island structure has a sea phase and plural island phases. The plural island phases exist (e.g., are dispersed) in the sea phase.

It is preferred that the sea phase contains the propylene polymer (A), and that the plural island phases each contain the thermoplastic polyurethane-based elastomer (B). When the resin composition further contains the polyethylene (D), it is preferred that the sea phase contains the propylene polymer (A), and that the plural island phases each contain an island phase of the thermoplastic polyurethane-based elastomer (B) and an island phase of the polyethylene (D).

The sea-island fibers have a fiber diameter of preferably 50 $\mu$m or less, more preferably 40 $\mu$m or less, still more preferably 30 $\mu$m or less, particularly preferably 28 $\mu$m or less. The smaller the fiber diameter, the superior is the flexibility of the nonwoven fabric. From the viewpoint of the ease of handling, the fiber diameter of the sea-island fibers is preferably 10 $\mu$m or more. The sea-island fibers may be long fibers (staples) or short fibers (filaments). A cross-sectional shape of the sea-island fibers is not particularly limited, and examples thereof include a circular shape, an elliptical shape, and an irregular shape.

(1.1.1.2.1) Island Phases of Thermoplastic Polyurethane-Based Elastomer (B)

When a cross-section of the fibers which is perpendicular to an axial direction is observed under a transmission electron microscope in a viewing area of 25.5 $\mu$m$^2$, it is preferred that island phases containing the thermoplastic polyurethane-based elastomer (B) have an average diameter of 0.095 $\mu$m or less, and that the number of the island phases containing the thermoplastic polyurethane-based elastomer (B) is 200 or more. Thereby, the spinnability can be improved.

A magnification rate of the transmission electron microscope is not particularly limited, and may be adjusted as appropriate such that the size and the number of the island phases can be measured at a cross-section of the fibers. For example, the magnification rate of the transmission electron microscope may be $\times$40,000.

Methods of measuring each of the average diameter and the number of the island phases containing the thermoplastic polyurethane-based elastomer (B) are the same as the methods described below in the section of Examples.

From the viewpoint of obtaining both satisfactory tensile strength and satisfactory air permeability in the spun-bonded nonwoven fabric, the average diameter of the island phases containing the thermoplastic polyurethane-based elastomer (B) is more preferably from 0.070 $\mu$m to 0.093 $\mu$m, still more preferably from 0.075 $\mu$m to 0.090 $\mu$m, particularly preferably from 0.078 $\mu$m to 0.087 $\mu$m.

From the viewpoint of obtaining both satisfactory tensile strength and satisfactory air permeability in the spun-bonded nonwoven fabric, the number of the island phases containing the thermoplastic polyurethane-based elastomer (B) is more preferably from 210 to 800, still more preferably from 215 to 700, particularly preferably from 220 to 650.

A ratio of the area of the island phases with respect to a total area of a cross-section of the sea-island fibers (hereinafter, referred to as "island phase area ratio") is preferably from 1.0% to 20%. By controlling the island phase area ratio to be in this range, oriented crystallization of the propylene polymer (A) is likely to be suppressed inside the sea-island fibers. This allows the spun-bonded nonwoven fabric to have superior extensibility and superior spinnability.

(1.1.1.2.2) Island Phases of Polyethylene (D)

In cases where the resin composition further contains the polyethylene (D), when a cross-section of the fibers which is perpendicular to an axial direction is observed under a transmission electron microscope in a viewing area of 25.5 $\mu$m$^2$, it is preferred that island phases containing the polyethylene (D) have an average diameter of 0.11 $\mu$m or less, and that the number of the island phases containing the polyethylene (D) is 150 or more. Thereby, not only the spinnability can be improved, but also the maximum elongation can be improved.

A magnification rate of the transmission electron microscope is not particularly limited, and may be adjusted as appropriate such that the size and the number of the island phases can be measured at a cross-section of the fibers. For example, the magnification rate of the transmission electron microscope may be $\times$40,000.

Methods of measuring each of the average diameter and the number of the island phases containing the polyethylene (D) are the same as the methods described below in the section of Examples.

From the viewpoint of obtaining both satisfactory tensile strength and satisfactory air permeability in the spun-bonded nonwoven fabric and the viewpoint of improving the maximum elongation, the average diameter of the island phases containing the polyethylene (D) is more preferably from 0.06 $\mu$m to 0.11 $\mu$m, still more preferably from 0.07 $\mu$m to 0.105 $\mu$m, particularly preferably from 0.08 $\mu$m to 0.105 $\mu$m.

From the viewpoint of obtaining both satisfactory tensile strength and satisfactory air permeability in the spun-bonded nonwoven fabric and the viewpoint of improving the maximum elongation, the number of the island phases containing the polyethylene (D) is more preferably from 150 to 400, still more preferably from 160 to 350, particularly preferably from 165 to 300.

(1.1.1.3) Forms, etc.

The fibers contained in the spun-bonded nonwoven fabric may be in the form of solid fibers or hollow fibers. From the viewpoint of reducing the fineness while maintaining the strength of the spun-bonded nonwoven fabric, the fibers contained in the spun-bonded nonwoven fabric are preferably solid fibers. As for a method of producing hollow fibers, reference can be made as appropriate to any method described in a known document.

A form of the fibers contained in the spun-bonded nonwoven fabric is not particularly limited, and the fibers may be in the form of composite fibers or monocomponent fibers. The composite fibers preferably contain two or more kinds of thermoplastic resins as constituents.

Examples of the composite fibers include composite fibers of a core-sheath type, a side-by-side type, a sea-island type, etc. The core-sheath-type composite fibers may each have a core portion and a sheath portion, and may be either concentric core-sheath-type composite fibers or eccentric core-sheath-type composite fibers. In the eccentric core-sheath-type composite fibers, the core portion may or may not be exposed to the surface.

The fibers contained in the spun-bonded nonwoven fabric may be crimped fibers or non-crimped fibers. The non-crimped fibers may be, for example, eccentric core-sheath-type crimped composite fibers.

(1.1.2) Resin Composition

The fibers are formed of a resin composition.

The resin composition contains a propylene polymer (A), a thermoplastic polyurethane-based elastomer (B), and a polymer (C). The propylene polymer (A), the thermoplastic polyurethane-based elastomer (B), and the polymer (C) will each be described below in detail.

It is preferred that: a content of the propylene polymer (A) is from 70.0% by mass to 97.0% by mass with respect to a total amount of the resin composition; a content of the thermoplastic polyurethane-based elastomer (B) is from 2.0% by mass to 20.0% by mass with respect to the total amount of the resin composition; and a content of the polymer (C) is from 1.0% by mass to 10.0% by mass with respect to the total amount of the resin composition. Thereby, not only the spinnability can be improved, but also satisfactory tensile strength and satisfactory air permeability can both be obtained in the spun-bonded nonwoven fabric.

(1.1.2.1) Propylene Polymer (A)

The resin composition contains a propylene polymer (A).

The propylene polymer (A) contains a structural unit derived from propylene as a main component. Specifically, the propylene polymer (A) contains a propylene homopolymer, and a copolymer of propylene and an $\alpha$-olefin other than propylene (this copolymer is hereinafter also referred to as "propylene/$\alpha$-olefin copolymer"). The propylene polymer (A) may be, for example, a propylene homopolymer or a propylene/$\alpha$-olefin copolymer, or may contain both of a propylene homopolymer and a propylene/$\alpha$-olefin copolymer.

In the resin composition, the propylene polymer (A) may be used singly, or in combination of two or more kinds thereof.

The propylene/$\alpha$-olefin copolymer is, for example, preferably a copolymer of propylene and one or more $\alpha$-olefins having from 2 to 10 carbon atoms other than propylene, more preferably a copolymer of propylene and one or more $\alpha$-olefins having from 2 to 8 carbon atoms other than propylene.

From the viewpoint of obtaining excellent flexibility, specific examples of a preferred $\alpha$-olefin copolymerized with propylene include ethylene, 1-butene, 1-pentene, 1-hexene, 1-octene, 1-decene, 3-methyl-1-butene, 3-methyl-1-pentene, 3-ethyl-1-pentene, 4-methyl-1-pentene, and 4-methyl-1-hexene.

Examples of the propylene/$\alpha$-olefin copolymer include propylene-ethylene copolymers, and propylene-ethylene-1-butene copolymers. The content of a structural unit derived from an $\alpha$-olefin in the propylene/$\alpha$-olefin copolymer is not particularly limited, and it is preferably from 1% by mole to 10% by mole, more preferably from 1% by mole to 5% by mole. In the resin composition, the propylene polymer (A) may be used singly, or in combination of two or more kinds thereof.

The melting point (Tm) of the propylene polymer (A) is not particularly limited, and it is preferably from 120°C to 165°C, more preferably from 130°C to 165°C, still more preferably from 150°C to 165°C.

When the propylene polymer (A) is a propylene homopolymer, the melting point (Tm) of the propylene polymer (A) is preferably 140°C or higher, more preferably 155°C or higher, still more preferably from 157°C to 165°C.

When the propylene polymer (A) is a propylene-$\alpha$-olefin copolymer, the melting point (Tm) of the propylene polymer (A) is preferably 153°C or lower, more preferably from 125°C to 150°C.

In the disclosure, the melting point (Tm) can be measured as follows by differential scanning calorimetry (DSC). Using DSC PYRIS 1 manufactured by PerkinElmer Co., Ltd. or DSC7020 manufactured by SII NanoTechnology Inc. as a differential scanning calorimeter (DSC), a sample (about 5 mg) is heated in a nitrogen atmosphere (20 ml/min) from room temperature to 200°C at a rate of 10°C/min, maintained at this temperature for 5 minutes, subsequently cooled to -50°C at a rate of 10°C/min, and then maintained at -50°C for 5 minutes, after which the sample is heated to 200°C at a rate of 10°C/min for the second time, and the melting point (Tm) is calculated from the crystal melting peak top in this second heating process. It is noted here that, when plural crystal melting peaks are observed, the peak on the highest temperature side is defined as the melting point (Tm).

From the viewpoint of obtaining both satisfactory tensile strength and satisfactory air permeability in the spun-bonded nonwoven fabric, the propylene polymer (A) preferably contains a propylene homopolymer having a melting point (Tm) of 140°C or higher, more preferably is a propylene homopolymer having a melting point (Tm) of 140°C or higher.

The melt flow rate (MFR) of the propylene polymer (A) is not particularly limited as long as a spun-bonded nonwoven fabric can be produced; however, from the viewpoint of obtaining both satisfactory tensile strength and satisfactory air permeability in the spun-bonded nonwoven fabric, the melt flow rate (MFR) of the propylene polymer (A) is preferably from 10 g/10 min to 100 g/10 min, more preferably from 15 g/10 min to 90 g/10 min, still more preferably from 20 g/10 min to 80 g/10 min.

The melt flow rate of the propylene polymer (A) is measured by a method conforming to the ASTM standard D-1238. As for the measurement conditions, the melt flow rate of the propylene polymer (A) is measured at 230°C with a load of 2.16 kg.

The density of the propylene polymer (A) is not particularly limited as long as the propylene polymer (A) can be melt-spun, and it is preferably from 0.900 g/cm$^3$ to 0.945 g/cm$^3$, more preferably from 0.905 g/cm$^3$ to 0.940 g/cm$^3$.

The density of the propylene polymer (A) can be measured in accordance with JIS K7112 (density gradient tube method).

The propylene polymer (A) can be obtained by polymerizing raw material propylene using a known catalyst, such as a Ziegler-Natta catalyst or a metallocene catalyst. Particularly, in order to eliminate heterophilic binding, it is preferred to use a Ziegler-Natta catalyst. Thereby, high-stereoregularity polymerization can be performed.

As a method of polymerizing propylene, any known method may be employed, and examples thereof include: a method of performing the polymerization in an inert solvent, such as hexane, heptane, toluene, or xylene; a method of performing the polymerization in a liquid monomer; a method of adding a catalyst to a gaseous monomer and performing the polymerization in a gaseous state; and a combination of these methods.

The propylene polymer (A) may be a commercially available product as well.

From the viewpoint of obtaining both satisfactory strength and satisfactory air permeability in the spun-bonded nonwoven fabric, the content of the propylene polymer (A) is preferably from 70.0% by mass to 97.0% by mass, more preferably from 72.0% by mass to 94.0% by mass, still more preferably from 75.0% by mass to 90.0% by mass, with respect to the total amount of the resin composition.

(1.1.2.1.1) Biomass-Derived Propylene Polymer

As the thermoplastic polyurethane-based elastomer of the disclosure, a thermoplastic polyurethane-based elastomer containing a biomass-derived raw material (biomass-derived thermoplastic polyurethane-based elastomer) can be used as well. The biomass-derived thermoplastic polyurethane-based elastomer can be obtained by polymerizing propylene that is obtained by dehydration of isopropanol produced by fermentation from a biomass raw material mainly composed of a non-edible plant such as sorgo.

The term "biomass degree" used herein refers to a content ratio of biomass-derived carbon, which is determined by measuring radioactive carbon ($C^{14}$). Since carbon dioxide in the atmosphere contains $C^{14}$ at a certain ratio (about 105.5 pMC), the $C^{14}$ content in those plants (e.g., corn) that grow by absorbing carbon dioxide in the atmosphere is known to be also about 105.5 pMC. It is also known that fossil fuels contain hardly any $C^{14}$. Accordingly, the content ratio of biomass-derived carbon in a raw material can be determined by measuring the ratio of $C^{14}$ contained in all carbon atoms of the raw material.

For example, when the content of $C^{14}$ in a raw material is defined as PC14, the content ratio Pbio (%) of biomass-derived carbon in the raw material can be calculated by the following equation (1):

$$\text{Pbio (\%)} = \text{PC14}/105.5 \times 100 \qquad (1)$$

In other words, when all of raw materials of a polyurethane are derived from biomass, the content ratio of biomass-derived carbon is theoretically 100%; therefore, the biomass degree of the biomass-derived thermoplastic polyurethane-based elastomer is 100%. Further, since a fossil fuel-derived raw material contains hardly any $C^{14}$, the content ratio of biomass-derived carbon in a polyurethane produced only from the fossil fuel-derived raw material is 0%, and a fossil fuel-derived polyurethane thus has a biomass degree of 0%.

The biomass degree of the biomass-derived thermoplastic polyurethane-based elastomer used as a raw material of the spun-bonded nonwoven fabric of the disclosure is preferably 1% or higher.

Generally, a thermoplastic polyurethane-based elastomer contains, as raw materials, for example: a polymer glycol, such as a polyether polyol, a polyester polyol, or a polycarbonate polyol; a short-chain glycol (chain extender), such as an aliphatic glycol, an aromatic glycol, or an alicyclic glycol; and an isocyanate compound, such as an aromatic diisocyanate, an aliphatic diisocyanate, or an alicyclic diisocyanate. Examples of an isocyanate preferably used in the disclosure include: aliphatic isocyanates such as 1,5-pentamethylene diisocyanate-based polyisocyanate (trade name:

STABiO (registered trademark), manufactured by Mitsui Chemicals, Inc.); and aromatic isocyanates.

When raw materials of the spun-bonded nonwoven fabric of the disclosure include a so-called recycled polymer, the thermoplastic polyurethane-based elastomer used as a raw material of the spun-bonded nonwoven fabric of the disclosure may contain the recycled polymer obtained by recycling.

The term "recycled polymer" encompasses a polymer obtained by recycling of a waste polymer product, and a recycled polymer can be produced by, for example, the method disclosed in DE 102019127827 (A1). The recycled polymer may also contain a marker with which the polymer can be identified as a product of recycling.

(1.1.2.2) Thermoplastic Polyurethane-Based Elastomer (B)

The resin composition contains a thermoplastic polyurethane-based elastomer (B).

The thermoplastic polyurethane-based elastomer (B) may have, for example, a hard segment and a soft segment that is amorphous and has a low glass transition temperature. In the hard segment, at least a polyurethane forms pseudo-crosslinks by physical aggregation. The soft segment is formed by a polymer such as a block copolymer. The block copolymer is composed of a polycarbonate-based polyol, an ether-based polyol, a caprolactone-based polyester, an adipate-based polyester, or the like.

Specific examples of the thermoplastic polyurethane-based elastomer (B) include the urethane-based thermoplastic elastomers (TPU) prescribed in JIS K6418:2007.

The thermoplastic polyurethane-based elastomer (B) has a higher density than a polyolefin, and can improve the fiber strength of the spun-bonded nonwoven fabric. This enables to maintain the strength of the spun-bonded nonwoven fabric itself even with a reduction in the amount of the fibers per unit volume of the spun-bonded nonwoven fabric (air permeability-improving factor). A higher content of the thermoplastic polyurethane-based elastomer (B) leads to a further improvement in the strength of the spun-bonded nonwoven fabric.

The melting point (Tm) of the thermoplastic polyurethane-based elastomer (B) is a value measured using a differential scanning calorimeter (DSC). Specifically, in a melting endothermic curve obtained with the use of a differential scanning calorimeter (DSC-7, manufactured by PerkinElmer Co., Ltd.) by maintaining 5 mg of a sample in a nitrogen atmosphere at -100°C for 5 minutes and subsequently raising the temperature at a rate of 10°C/min, the temperature of the top of a peak observed on the highest temperature side is the melting point (Tm) of the thermoplastic polyurethane-based elastomer (B).

The melting point (Tm) of the thermoplastic polyurethane-based elastomer (B) is not particularly limited, and it is preferably 165°C or lower, more preferably 163°C or lower.

The amount of heat of fusion of the thermoplastic polyurethane-based elastomer (B) is a value measured using a differential scanning calorimeter (DSC). Specifically, in a melting endothermic curve obtained with the use of a differential scanning calorimeter (DSC-7, manufactured by PerkinElmer Co., Ltd.) by maintaining 5 mg of a sample in a nitrogen atmosphere at -100°C for 5 minutes and subsequently raising the temperature at a rate of 10°C/min, the amount of heat of fusion determined from the highest endothermic peak is the amount of heat of fusion of the thermoplastic polyurethane-based elastomer (B).

The amount of heat of fusion of the thermoplastic polyurethane-based elastomer (B) is not particularly limited, but it is preferably 14.0 mJ/mg or less, more preferably 12.0 mJ/mg or less.

The thermoplastic polyurethane-based elastomer (B) has a solidification start temperature of preferably from 65°C to 195°C, more preferably from 75°C to 195°C, still more preferably from 85°C to 195°C.

When the solidification start temperature is 65°C or higher, it is possible to suppress molding defects such as fusion between fibers, thread breakage, resin lumps, etc. When obtaining spunbond nonwoven fabric, and spunbond nonwoven fabric molded during heat embossing, it is possible to prevent it from winding around the embossing roller. The resulting spun-bonded nonwoven fabric has low stickiness and can thus be suitably used as a material coming into contact with the skin, such as a clothing material, a hygienic material, or a material of sporting goods. Meanwhile, by controlling the solidification start temperature to be 195°C or lower, the forming processability can be improved. It is noted here that formed fibers tend to have a higher solidification start temperature than that of the thermoplastic polyurethane-based elastomer (B) used as a raw material of the fibers.

The solidification start temperature of the thermoplastic polyurethane-based elastomer (B) is a value measured using a differential scanning calorimeter (DSC). Specifically, the solidification start temperature of the thermoplastic polyurethane-based elastomer (B) is the start temperature of an exothermic peak attributed to solidification of the thermoplastic polyurethane-based elastomer (B) that occurs when the thermoplastic polyurethane-based elastomer (B) is heated to 230°C at a rate of 10°C/min, maintained at 230°C for 5 minutes, and then cooled at a rate of 10°C/min.

In order to adjust the solidification start temperature of the thermoplastic polyurethane-based elastomer (B) to be 65°C or higher, it is necessary to not only select compounds each having an optimum chemical structure as a polyol, an isocyanate compound, and a chain extender that are used as raw materials of the thermoplastic polyurethane-based elastomer (B), but also adjust the amount of hard segment. The "amount of hard segment" refers to a value in mass percent (% by mass) which is calculated by dividing a total mass of the isocyanate compound and the chain extender that are used in the production of the thermoplastic polyurethane-based elastomer (B) by a total amount of the polyol, the isocyanate compound, and the chain extender, and then multiplying the resulting value by 100. The amount of hard segment is preferably from 20% by mass to 60% by mass, more preferably from 22% by mass to 50% by mass, still more preferably from 25% by mass to 48% by mass.

The number of particles of polar solvent-insoluble components in the thermoplastic polyurethane-based elastomer (B) is preferably 3,000,000 (3,000,000/g) or less, more preferably 2,500,000/g or less, still more preferably 2,000,000/g or less, with respect to 1 g of the thermoplastic polyurethane-based elastomer (B).

The "polar solvent-insoluble components in the thermoplastic polyurethane-based elastomer (B)" are mainly aggregates (e.g., fisheyes and gels) generated during the production of the thermoplastic polyurethane-based elastomer (B).

By controlling the number of particles of the polar solvent-insoluble components to be 3,000,000/g or less, the occurrence of problems such as an increase in the fiber diameter distribution and fiber breakage in spinning can be further suppressed within the solidification start temperature range of the thermoplastic polyurethane-based elastomer (B).

Causes of polar solvent insoluble components include: components derived from the hard segment aggregates of the thermoplastic polyurethane-based elastomer (B); components generated by crosslinking of the hard segment and/or the soft segment via allophanate bonds, biuret bonds, etc.; and components generated by a chemical reaction between raw materials constituting the thermoplastic polyurethane-based elastomer (B).

The number of particles of the polar solvent-insoluble components is a value determined by dissolving the thermoplastic polyurethane-based elastomer (B) in a dimethylacetamide solvent and measuring the resulting insoluble components using a particle size distribution analyzer that utilizes a pore electrical resistance method and is fitted with a $100\text{-}\mu\text{m}$ aperture. By fitting the analyzer with a $100\text{-}\mu\text{m}$ aperture, the number of particles of from $2\ \mu\text{m}$ to $60\ \mu\text{m}$ can be measured in terms of non-crosslinked polystyrene.

The thermoplastic polyurethane-based elastomer (B) having a low content of the polar solvent-insoluble components can be obtained by filtering the resultant after performing a polymerization reaction of the polyol, the isocyanate compound, and the chain extender.

From the viewpoint of suppressing the incorporation of air bubbles into a strand and the occurrence of fiber breakage during the forming of the spun-bonded nonwoven fabric using a large-sized spunbond forming machine, the thermoplastic polyurethane-based elastomer (B) has a water content of preferably 350 ppm or less, more preferably 300 ppm, or less, still more preferably 150 ppm or less.

In the thermoplastic polyurethane-based elastomer (B), from the viewpoint of obtaining both satisfactory tensile strength and satisfactory air permeability in the spun-bonded nonwoven fabric, a sum (a) of the heat of fusion and a sum (b) of the heat of fusion preferably satisfies the relationship of the following formula (B1), more preferably satisfies the relationship of the following formula (B2), still more preferably satisfies the relationship of the following formula (B3). The sum (a) of the heat of fusion and the sum (b) of the heat of fusion are each measured using a differential scanning calorimeter (DSC). The sum (a) of the heat of fusion is determined from an endothermic peak having a peak temperature in a range of from 90°C to 140°C. The sum (b) of the heat of fusion is determined from an endothermic peak having a peak temperature in a range of higher than 140°C but 220°C or lower.

$$\text{Formula (B1): } a/(a+b) \le 0.8$$

$$\text{Formula (B2): } a/(a+b) \le 0.7$$

$$\text{Formula (B3): } a/(a+b) \le 0.6$$

The value of "$a/(a+b)$" means a ratio of the heat of fusion of a hard domain of the thermoplastic polyurethane-based elastomer (B).

In the disclosure in which the spun-bonded nonwoven fabric can attain both satisfactory tensile strength and satisfactory air permeability, when the ratio ($a/(a+b)$) of the heat of fusion of the hard domain of the thermoplastic polyurethane-based elastomer (B) is 0.8 or lower, a lower limit of the ratio ($a/(a+b)$) of the heat of fusion of the hard domain of the thermoplastic polyurethane-based elastomer (B) is preferably about 0.1.

The thermoplastic polyurethane-based elastomer (B) has a melt viscosity of preferably from 100 Pa-s to 3,000 Pa-s, more preferably from 200 Pa·s to 2,000 Pa·s, still more preferably from 900 Pa·s to 1,600 Pa·s.

The melt viscosity is a value measured by CAPIROGRAPH (manufactured by Toyo Seiki Seisaku-sho, Ltd., nozzle length: 30 mm, diameter: 1 mm). The melt viscosity measurement conditions are a temperature of 200°C and a shear rate of 100 sec[-1].

The thermoplastic polyurethane-based elastomer (B) having the above-described properties can be obtained, for example, by the production method disclosed in Japanese Patent Application Laid-Open (JP-A) No. 2004-244791.

A spun-bonded nonwoven fabric formed using the thermoplastic polyurethane-based elastomer (B) has excellent texture and can thus be suitably used in applications that come into contact with the skin, such as hygienic materials. The thermoplastic polyurethane-based elastomer (B) is industrially preferable for filtration of impurities, etc., when the filter arranged inside an extruder, it is likely difficult to clog, and thus reduces the frequency of adjustment and maintenance of equipment.

In the resin composition, the thermoplastic polyurethane-based elastomer (B) may be used singly, or in combination of two or more kinds thereof.

From the viewpoint of obtaining both satisfactory tensile strength and satisfactory air permeability in the spun-bonded nonwoven fabric, the content of the thermoplastic polyurethane-based elastomer (B) is preferably from 2.0% by mass to 20.0% by mass, more preferably from 2.5% by mass to 19.0% by mass, still more preferably from 3.0% by mass to 18.0% by mass, with respect to the total amount of the resin composition.

(1.1.2.3) Polymer (C)

The resin composition contains a polymer (C).

The polymer (C) functions as a compatibilizer that compatibilizes the propylene polymer (A) and the thermoplastic polyurethane-based elastomer (B). By incorporating the polymer (C) into the resin composition, blended components can be sufficiently dissolved in the resin composition while the resin composition contains the propylene polymer (A) and the thermoplastic polyurethane-based elastomer (B).

The polymer (C) is modified with a polar group. In other words, the polymer (C) has a main chain and a polar group bound to the main chain. In the resin composition, the polymer (C) may be used singly, or in combination of two or more kinds thereof.

(1.1.2.3.1) Polar Group

The polar group is at least one selected from oxygen-containing groups, nitrogen-containing groups, sulfur-containing groups, or phosphorus-containing groups.

Examples of the oxygen-containing groups include alkoxy groups, aryloxy groups, ester groups, ether groups, acyl groups, carboxyl groups, carbonate groups, hydroxy groups, peroxy groups, carboxylic acid anhydride groups (-CO-O-CO-), and carboxyl group substitutes.

Examples of the nitrogen-containing groups include amino groups, alkylamino groups, arylamino groups, alkylarylamino groups, imino groups, alkylimino groups, arylimino groups, imide groups, substituted imide groups, amide groups, substituted amide groups, hydrazino groups, hydrazono groups, nitro groups, nitroso groups, cyano groups, isocyano groups, cyanate groups, amidino groups, diazo groups, amino groups, alkylamino groups, arylamino groups, and ammonium salt of an alkylarylamino groups.

The alkylamino groups is preferably dimethylamino groups or ethylmethylamino groups. The arylamino group is preferably diphenylamino groups. The alkylimino groups are preferably methylimino groups, ethylimino groups, propylimino groups, or butylimino groups. The arylimino groups are preferably phenylimino groups. The substituted imide groups are preferably acetimide groups or benzimide groups. The substituted amide groups are preferably acetamide groups, *N*-methyl acetamide groups, or *N*-methylbenzamide groups.

Examples of the sulfur-containing groups include mercapto groups, thioester groups, dithioester groups, alkylthio groups, arylthio groups, thioacyl groups, thioether groups, thiocyanate groups, isothiocyanate groups, sulfonate groups, sulfonamide groups, thiocarboxyl groups, dithiocarboxyl groups, sulfo groups, sulfonyl groups, sulfinyl groups, and sulfenyl groups.

The alkylthio groups are preferably methylthio groups or ethylthio groups. The arylthio groups are preferably phenylthio groups, methylphenylthio groups, or naphthylthio groups. The thioester groups are preferably acetylthio groups, benzoylthio groups, methylthiocarbonyl groups, or phenylthiocarbonyl groups. The sulfonate groups are preferably methyl sulfonate groups, ethyl sulfonate groups, or phenyl sulfonate groups. The sulfonamide groups are preferably phenylsulfonamide groups, *N*-methylsulfonamide groups, or *N*-methyl-*p*-toluenesulfonamide.

Examples of the phosphorus-containing groups include phosphide groups, phosphoryl groups, thiophosphoryl groups, and phosphate groups.

The polar group preferably contains at least one of oxygen-containing groups or nitrogen-containing groups. Thereby, not only the spinnability can be improved, but also satisfactory tensile strength and satisfactory air permeability can both be obtained in the spun-bonded nonwoven fabric.

The polar group preferably contains at least one of amino groups, carboxyl groups, carboxylic acid anhydride groups, or ester groups. Thereby, not only the spinnability can be improved, but also satisfactory tensile strength and satisfactory air permeability can both be obtained in the spun-bonded nonwoven fabric.

From the viewpoint of obtaining both satisfactory tensile strength and satisfactory air permeability in the spun-bonded nonwoven fabric, the polar group more preferably contains at least one of amino groups and carboxylic acid anhydride groups, or particularly preferably contains amino groups.

(1.1.2.3.2) Main Chain

The main chain of the polymer (C) is, for example, a polyolefin or a polystyrene. Examples of the polystyrene include hydrogenated styrene-based block copolymers.

Examples of the polyolefin include: $\alpha$-olefins having from 2 to 20 carbon atoms, preferably from 2 to 6 carbon atoms, such as ethylene, propylene, 1-butene, 1-pentene, 1-hexene, 1-heptene, 1-octene, 1-decene, 3-methyl-1-butene, 3-methyl-1-pentene, 3-ethyl-1-pentene, and 4-methyl-1-pentene; and homo- or co-polymers of linear or cyclic polyenes, such as cyclopentene, cyclohexene, 1,4-hexadiene, 1,5-hexadiene, divinylbenzene, 1,3-cyclopentadiene, 1,3-cyclohexadiene, and 5-vinyl-2-norbornene.

The main chain of the polymer (C) preferably contains a polyolefin or a hydrogenated styrene-based block copolymer. Thereby, not only the spinnability can be improved, but also satisfactory tensile strength and satisfactory air permeability can both be obtained in the spun-bonded nonwoven fabric.

The polymer (C) may be a commercially available product.

Examples of a commercially available product of a hydrogenated styrene-based block copolymer containing carboxylic acid anhydride groups or amino groups as a polar group include TUFTEC M Series (manufactured by Asahi Kasei Corporation).

Examples of a commercially available product of a polyolefin containing carboxylic acid anhydride groups as a polar group include UMEX (manufactured by Sanyo Chemical Industries, Inc.) and ADMER (manufactured by Mitsui Chemicals, Inc.).

Examples of a commercially available product of a polyolefin containing carboxyl groups as a polar group include NUCREL (manufactured by Dow-Mitsui Polychemicals Co., Ltd.).

Examples of a commercially available product of a polyolefin containing ester groups as a polar group include ACRYFT (manufactured by Sumitomo Chemical Co., Ltd.).

From the viewpoint of obtaining both satisfactory tensile strength and satisfactory air permeability in the spun-bonded nonwoven fabric, the content of the polymer (C) is preferably from 1.0% by mass to 10.0% by mass, more preferably from 1.5% by mass to 9.0% by mass, still more preferably from 2.0% by mass to 7.0% by mass, with respect to the total amount of the resin composition.

(1.1.2.4) Polyethylene (D)

The resin composition may also contain a polyethylene (D). Thereby, the extensibility of the spun-bonded nonwoven fabric can be improved.

The polyethylene (D) contains a structural unit derived from ethylene. Examples of the polyethylene (D) include ethylene homopolymers, such as high-pressure low-density polyethylenes, linear low-density polyethylenes (LLDPE), and high-density polyethylenes (HDPE). In the resin composition, the polyethylene (D) may be used singly, or in combination of two or more kinds thereof.

The resin composition preferably further contain a polyethylene (D) having a density of from 0.941 g/cm$^3$ to 0.970 g/cm$^3$. Thereby, not only satisfactory tensile strength and satisfactory air permeability can both be obtained but also the maximum elongation can be improved in the spun-bonded nonwoven fabric.

From the viewpoint of further improving the extensibility (the property of the maximum elongation) of the spun-bonded nonwoven fabric, the density of the polyethylene (D) is preferably from 0.941 g/cm$^3$ to 0.970 g/cm$^3$, more preferably from 0.945 g/cm$^3$ to 0.960 g/cm$^3$.

From the viewpoint of obtaining both satisfactory tensile strength and satisfactory air permeability in the spun-bonded nonwoven fabric and improving the maximum elongation, a content of the polyethylene (D) is preferably from 1.0% by mass to 10.0% by mass, more preferably from 3.0% by mass to 9.0% by mass, with respect to the total amount of the resin composition.

(1.1.2.5) Low-Molecular-Weight Olefin Polymer (E)

The resin composition may also contain a low-molecular-weight olefin polymer (E).

The low-molecular-weight olefin polymer (E) is an olefin-based polymer that has a weight-average molecular weight of from 500 to 30,000 and does not correspond to any of the propylene polymer (A), the thermoplastic polyurethane-based elastomer (B), the polymer (C), and the polyethylene (D).

In the resin composition, the low-molecular-weight olefin polymer (E) may be used singly, or in combination of two or more kinds thereof.

When the resin composition contains the polyethylene (D) and the low-molecular-weight olefin polymer (E), the dispersibility of the propylene polymer (A) and the polyethylene (D) is improved. As a result, the extensibility and the spinnability of the spun-bonded nonwoven fabric tend to be improved.

The low-molecular-weight olefin polymer (E) will now be described for cases where the resin composition further contains the polyethylene (D).

The low-molecular-weight olefin polymer (E) may contain a single kind of olefin-derived structural unit, or may contain two or more kinds of olefin-derived structural units.

The low-molecular-weight olefin polymer (E) is a wax-like polymer. The low-molecular-weight olefin polymer (E) preferably has a lower weight-average molecular weight (Mw) than those of the propylene polymer (A) and the polyethylene (D).

The weight-average molecular weight (Mw) of the low-molecular-weight olefin polymer (E) is from 500 to 30,000.

As long as the weight-average molecular weight (Mw) of the low-molecular-weight olefin polymer (E) is in this range, the dispersibility of the propylene polymer (A) and the polyethylene (D) is further improved. As a result, the spun-bonded nonwoven fabric exhibits superior extensibility and spinnability.

The weight-average molecular weight (Mw) of the low-molecular-weight olefin polymer (E) is 30,000 or less, preferably less than 15,000, more preferably 10,000 or less, still more preferably 6,000 or less, particularly preferably less than 6,000, further preferably 5,000 or less, still further preferably 3,000 or less, yet still further preferably 2,000 or less, further more preferably 1,500 or less.

The weight-average molecular weight (Mw) of the low-molecular-weight olefin polymer (E) is not less than 500, preferably not less than 700, more preferably not less than 1,000.

For the measurement of the weight-average molecular weight (Mw) of the low-molecular-weight olefin polymer (E), gel permeation chromatography (GPC) is used. The GPC measurement conditions are preferably as described below. The weight-average molecular weight (Mw) of the low-molecular-weight olefin polymer (E) is determined, for example, based on the below-described conversion method using a calibration curve that is prepared using a commercially available monodisperse standard polystyrene.

[Measurement Conditions]

Columns: TSKgel GMH6-HT $\times$ 2 (manufactured by Tosoh Corporation) and TSKgel GMH6-HTL column $\times$ 2 (manufactured by Tosoh Corporation)
Apparatus: gel permeation chromatograph ALLIANCE GPC 2000 (manufactured by Waters Corporation)
Solvent: *o*-dichlorobenzene
Flow rate: 1.0 mL/min
Sample: 0.15 mg/mL, *o*-dichlorobenzene solution
Temperature: 140°C
Molecular weight conversion: polyethylene (PE) conversion/general calibration method
For the calculation of general calibration, the following coefficients of the Mark-Houwink viscosity equation were used.
Coefficient of polystyrene (PS): KPS = 1.38 $\times$ 10$^{-4}$, aPS = 0.70
Coefficient of polyethylene (PE): KPE = 5.06 $\times$ 10$^{-4}$, aPE = 0.70

The low-molecular-weight olefin polymer (E) has a softening point of preferably from 90°C to 145°C, more preferably from 90°C to 135°C, still more preferably from 100°C to 125°C.

The softening point of the low-molecular-weight olefin polymer (E) is measured in accordance with JIS K2207.

The density of the low-molecular-weight olefin polymer (E) is not particularly limited, and it is preferably from 0.890 g/cm$^3$ to 0.980 g/cm$^3$. When the density of the low-molecular-weight olefin polymer (E) is in this range, the spun-bonded nonwoven fabric exhibits superior extensibility.

The density of the low-molecular-weight olefin polymer (E) is more preferably 0.910 g/cm$^3$ or higher, still more preferably 0.920 g/cm$^3$ or higher.

The density of the low-molecular-weight olefin polymer (E) is more preferably 0.960 g/cm$^3$ or lower, still more preferably 0.940 g/cm$^3$ or lower.

A difference between the density of the low-molecular-weight olefin polymer (E) and that of the propylene polymer (A) is not particularly limited, and it is preferably less than 0.35 g/cm$^3$, more preferably less than 0.20 g/cm$^3$, still more preferably less than 0.15 g/cm$^3$.

When the difference between the density of the low-molecular-weight olefin polymer (E) and that of the propylene polymer (A) is in this range, the spun-bonded nonwoven fabric exhibits superior extensibility.

The reason for this is not clear; however, it isconsiderd as follows. When the density of the low-molecular-weight olefin polymer (E) and that of the propylene polymer (A) are in the above-described respective ranges, for example, the polyethylene (D) is easily dispersed in the propylene polymer (A) via (with the work of) the low-molecular-weight olefin polymer (E). That is, the low-molecular-weight olefin polymer (E) effectively acts as a compatibilizer of the propylene polymer (A) and the polyethylene (D). Thus, the dispersibility of the propylene polymer (A) and the polyethylene (D) is improved. It is considered that, as a result, the extensibility of the spun-bonded nonwoven fabric is improved.

The low-molecular-weight olefin polymer (E) is an olefin homopolymer, or an olefin copolymer formed of two or more kinds of olefins.

Particularly, the low-molecular-weight olefin polymer (E) may be formed of ethylene and an $\alpha$-olefin having from 3 to 20 carbon atoms.

The number of carbon atoms of the $\alpha$-olefin is preferably from 3 to 8, more preferably from 3 or 4.

As long When the number of carbon atoms of the $\alpha$-olefin is in this range, the extensibility and the spinnability of the spun-bonded nonwoven fabric are further improved. The reason for this is not clear; however, it is considered as follows.

When the number of carbon atoms of the $\alpha$-olefin is in the above-described range, for example, the polyethylene (D) is easily dispersed in the propylene polymer (A) via the low-molecular-weight olefin polymer (E). In other words, the low-molecular-weight olefin polymer (E) effectively acts as a compatibilizer of the propylene polymer (A) and the polyethylene (D). Thus, the uniformity of the propylene polymer (A) and the polyethylene (D) is improved. It is considered that, as a result, the properties of the spun-bonded nonwoven fabric, such as elongation, are improved.

In the resin composition, the low-molecular-weight olefin polymer (E) may be used singly, or in combination of two or more kinds thereof.

A method of producing the low-molecular-weight olefin polymer (E) is not particularly limited, and examples thereof include a first production method and a second production method. The first production method refers to a production method based on polymerization of a commonly used low-molecular-weight polymer. The second production method refers to a method of reducing the molecular weight of a high-molecular-weight ethylene polymer by thermal degradation.

The resulting low-molecular-weight olefin polymer (E) may be purified by, for example, a solvent fractionation method of performing fractionation based on a difference in solubility in a solvent, or a distillation method.

Examples of the first production method include production methods using a Ziegler-Natta catalyst, a metallocene catalyst, or the like. Examples of a production method using a metallocene catalyst or the like include the production methods disclosed in JP-A No. H08-239414, WO 2007/114102, etc.

The low-molecular-weight olefin polymer (E) may be a commercially available product. Examples thereof include "Hi-WAX (registered trademark) 320P", "EXCEREX (registered trademark) 30200B", "Hi-WAX (registered trademark) 100P", and "Hi-WAX (registered trademark) 110P", which are manufactured by Mitsui Chemicals, Inc.

The content of the low-molecular-weight olefin polymer (E) is preferably from 0.1% by mass to 5.0% by mass with respect to the total amount of the resin composition.

From the viewpoint of allowing a sufficient amount of the low-molecular-weight olefin polymer (E) to exist at the interfaces between the sea phase and the island phases so as to improve the dispersibility of the propylene polymer (A) and the polyethylene (D), the content of the low-molecular-weight olefin polymer (E) is more preferably not less than 0.2% by mass, still more preferably not less than 1.0% by mass, particularly preferably not less than 1.5% by mass, with respect to the total amount of the resin composition.

From the viewpoint of suppressing a reduction in the strength of the fibers, the content of the low-molecular-weight olefin polymer (E) is more preferably 4.0% by mass or less, still more preferably 3.0% by mass or less, particularly preferably 2.5% by mass or less, with respect to the total amount of the resin composition.

(1.1.2.6) Other Polymer (F)

From the viewpoint of improving the extensibility of the fibers, the resin composition may also contain other polymer (F). This "other polymer (F)" refers to a polymer different from all of the propylene polymer (A), the thermoplastic polyurethane-based elastomer (B), the polymer (C), the polyethylene (D), and the low-molecular-weight olefin polymer (E).

Examples of the other polymer (F) include, but not particularly limited to, a polymer represented by the below-described (F1) (hereinafter, also referred to as "polymer (F1)"), and a polymer represented by the below-described (F2) (hereinafter, also referred to as "polymer (F2)").

(F1): a random copolymer of propylene and at least one selected from ethylene and $\alpha$-olefins having from 4 to 20 carbon atoms

(F2): a propylene homopolymer that satisfies the following (a) to (f) and has a melting point (Tm) of lower than 120°C:

(a) [mmmm] = from 20% by mole to 60% by mole,
(b)

$$[rrrr]/(1 - [mmmm]) \le 0.1,$$

(c) [rmrm] > 2.5% by mole,
(d)

$$[mm] \times [rr]/[mr]^2 \le 2.0,$$

(e) weight-average molecular weight (Mw) = from 10,000 to 200,000, and
(f) molecular weight distribution (Mw/Mn) < 4.

In (a) to (d), [mmmm] represents a mesopentad fraction; [rrrr] represents a racemic pentad fraction; [rmrm] represents a racemic-mesoracemic-mesopentad fraction; and [mm], [rr], and [mr] each represent a triad fraction.

(1.1.2.6.1) Polymer (F1)

The polymer (F1) is a random copolymer containing a structural unit derived from propylene, and a structural unit derived from at least one $\alpha$-olefin selected from ethylene and $\alpha$-olefins having from 4 to 20 carbon atoms. When the polymer (F1) is a random copolymer, the flexibility of the resulting spun-bonded nonwoven fabric tends to be improved without generating a sticky feelings.

The polymer (F1) is not particularly limited as long as it is a random copolymer containing the above-described structural units.

Examples of a structural unit copolymerizable with propylene include: structural units derived from ethylene; and structural units derived from an $\alpha$-olefin selected from $\alpha$-olefins having 4 or more carbon atoms, such as 1-butene, 1-pentene, 1-hexene, 1-octene, 1-decene, 3-methyl-1-butene, 3-methyl-1-pentene, 3-ethyl-1-pentene, 4-methyl-1-pentene, and 4-methyl-1-hexene. Thereamong, structural units derived from ethylene and structural units derived from an $\alpha$-olefin selected from $\alpha$-olefins having from 4 to 8 carbon atoms are preferred.

In the polymer (F1), a structural unit derived from an $\alpha$-olefin may be contained singly, or in combination of two or more kinds thereof.

Specific examples of the polymer (F1) include propylene-1-butene random copolymers, propylene-ethylene random copolymers, and propylene-ethylene-1-butene random copolymers.

A total ratio of the structural unit derived from propylene and the structural unit derived from an $\alpha$-olefin other than propylene, such as ethylene, in all structural units contained in the polymer (F1) is preferably 80% by mole or more, more preferably 85% by mole or more, still more preferably 90% by mole or more.

The polymer (F1) has a melting point (Tm) of preferably 100°C or higher, more preferably 130°C or higher, still more preferably 150°C or higher.

The melting point (Tm) of the polymer (F1) is defined as the top of a peak observed on the highest temperature side of a melting endothermic curve that is obtained using a differential scanning calorimeter (DSC) in which the polymer (F1) is maintained in a nitrogen atmosphere at -40°C for 5 minutes and the temperature is subsequently raised at a rate of 10°C/min. Specifically, the melting point (Tm) can be determined as the top of a peak observed on the highest temperature side of a melting endothermic curve obtained with the use of a differential scanning calorimeter (DSC-7, manufactured by PerkinElmer Co., Ltd.) by maintaining 5 mg of a sample in a nitrogen atmosphere at -40°C for 5 minutes and subsequently raising the temperature at a rate of 10°C/min.

The polymer (F1) has a crystallinity of preferably 15% or less, more preferably 10% or less, still more preferably 8% or less.

The crystallinity of the polymer (F1) is calculated from a melting heat curve attributed to melting of a main component in a melting endothermic curve that is obtained using a differential scanning calorimeter (DSC) in which the polymer (F1) is maintained in a nitrogen atmosphere at -40°C for 5 minutes and the temperature is subsequently raised at a rate of 10°C/min. Specifically, in a melting endothermic curve obtained with the use of a differential scanning calorimeter (DSC-7, manufactured by PerkinElmer Co., Ltd.) by maintaining 5 mg of a sample in a nitrogen atmosphere at -40°C for 5 minutes and subsequently raising the temperature at a rate of 10°C/min, the crystallinity of the polymer (F1) can be calculated from a melting heat curve attributed to melting of a main component, using the following equation:

$$\text{Crystallinity} = (\Delta H/\Delta H0) \times 100\ (\%)$$

In this equation, $\Delta H$ represents the amount of heat of fusion (J/g) determined from the melting heat curve attributed to melting of a main component of the polymer (F1), and $\Delta H0$ represents the amount of heat of fusion (J/g) of a perfect crystal of the main component. In other words, when the main component is ethylene, $\Delta H0$ is 293 J/g, while when the main component is propylene, $\Delta H0$ is 210 J/g.

The polymer (F1) has a tensile elastic modulus of preferably 100 MPa or less, more preferably 40 MPa or less, still more preferably 25 MPa or less.

The tensile elastic modulus of the polymer (F1) is measured by a method conforming to JIS K7161 (changed to JIS K7161-1:2014, corresponding to ISO 527-1:2012).

From the viewpoint of obtaining favorable spinnability and excellent stretching process suitability, the polymer (F1) has a melt flow rate of preferably from 1 g/10 min to 100 g/10 min, more preferably from 5 g/10 min to 100 g/10 min, still more preferably from 30 g/10 min to 70 g/10 min.

The melt flow rate of the polymer (F1) is measured by a method conforming to the ASTM Standard D-1238. As for the measurement conditions, the melt flow rate of the polymer (F1) is measured at 230°C with a load of 2.16 kg.

A ratio (Mw/Mn; molecular weight distribution) between the weight-average molecular weight (Mw) and the number-average molecular weight (Mn) of the polymer (F1) is preferably from 1.5 to 5.0 and, from the viewpoint of obtaining fibers having more favorable spinnability and particularly excellent fiber strength, this ratio (Mw/Mn) is more preferably from 1.5 to 3.0.

The Mw and the Mn of the polymer (F1) can be measured by GPC (gel permeation chromatography) in accordance with the below-described method.

(1.1.2.6.2) Polymer (F2)

The polymer (F2) is a propylene homopolymer that satisfies the below-described (a) to (f) and has a melting point (Tm) of lower than 120°C.

(1.1.2.6.2.1) [mmmm]

The polymer (F2) satisfies the following equation (a):

$$\text{Equation (a): } [mmmm] = \text{from } 20\% \text{ by mole to } 60\% \text{ by mole}$$

When the mesopentad fraction [mmmm] of the polymer (F2) is 20% by mole or more, the occurrence of stickiness is suppressed, and when the mesopentad fraction [mmmm] is 60% by mole or less, since the crystallinity is not excessively high, so that, favorable elastic recovery is obtained.

The mesopentad fraction [mmmm] of the polymer (F2) is preferably from 30% by mole to 50% by mole, more preferably from 40% by mole to 50% by mole.

The mesopentad fraction [mmmm] and the below-described racemic pentad fraction [rrrr] and racemic-mesoracemic-mesopentad fraction [rmrm] are a meso-fraction, a racemic fraction, and a racemic-mesoracemic meso-fraction, respectively, in a pentad unit in a polypropylene molecular chain, which are measured based on the signal of methyl groups in a $^{13}$C-NMR spectrum in accordance with the method proposed in "Macromolecules, 6, 925 (1973)" by A. Zambelli et al. An increase in the mesopentad fraction [mmmm] leads to a higher stereoregularity. The below-described triad fractions [mm], [rr], and [mr] are also determined by the above-described method.

It is noted here that the $^{13}$C-NMR spectrum can be measured using the following apparatus under the following conditions in accordance with the peak attribution proposed by A. Zambelli et al. in "Macromolecules, 8, 687 (1975)".

[Apparatus and Conditions]

Apparatus: JNM-EX400 $^{13}$C-NMR apparatus, manufactured by JEOL Ltd.

Method: proton complete decoupling
Concentration: 220 mg/mL
Solvent: a 90:10 (volume ratio) mixed solvent of 1,2,4-trichlorobenzene and deuterated benzene
Temperature: 130°C
Pulse width: 45°
Pulse interval: 4 seconds

Integration: 10,000 times

$$M = m/S \times 100$$

$$R = \gamma/S \times 100$$

$$S = P\beta\beta + P\alpha\beta + P\alpha\gamma$$

S: signal intensity of side-chain methyl carbon atoms of all propylene units
$P\beta\beta$: from 19.8 ppm to 22.5 ppm
$P\alpha\beta$: from 18.0 ppm to 17.5 ppm
$P\alpha\gamma$: from 17.5 ppm to 17.1 ppm
$\gamma$: racemic pentad chain: from 20.7 ppm to 20.3 ppm
$m$: mesopentad chain: from 21.7 ppm to 22.5 ppm

(1.1.2.6.2.2) [rrrr]/(1 - [mmmm])

The polymer (F2) satisfies the following equation (b):

$$\text{Equation (b): } [rrrr]/(1 - [mmmm]) \le 0.1$$

The value of [rrrr]/[1 - [mmmm] is determined from the above-described fractions of the pentad unit, and is an index that indicates the uniformity of the regularity distribution of the structural unit derived from propylene in the polymer (F2). When this value is large, the polymer (F2) is a mixture of a high-regularity polypropylene and an atactic polypropylene as in the case of a conventional polypropylene produced using an existing catalyst system, and causes stickiness.
When the polymer (F2) has a [rrrr]/(1 - [mmmm]) value of 0.1 or less, stickiness is suppressed in the resulting spun-bonded nonwoven fabric.
From this viewpoint, the value of [rrrr]/(1 - [mmmm]) is preferably 0.05 or less, more preferably 0.04 or less.

(1.1.2.6.2.3) [rmrm]

The polymer (F2) satisfies the following equation (c):

$$\text{Equation (c): } [rmrm] > 2.5\% \text{ by mole}$$

When the racemic-mesoracemic meso-fraction [rmrm] of the polymer (F2) exceeds 2.5% by mole, the randomness of the polymer (F2) is increased, so that the elastic recovery of the spun-bonded nonwoven fabric is further improved.
The racemic-mesoracemic meso-fraction [rmrm] of the polymer (F2) is preferably from 2.6% by mole to 10% by mole, more preferably from 2.7% by mole to 10% by mole.

(1.1.2.6.2.4) [mm] $\times$ [rr]/[mr]$^2$

The polymer (F2) satisfies the following equation (d):

$$\text{Equation (d): } [mm] \times [rr]/[mr]^2 \le 2.0$$

The value of [mm] $\times$ [rr]/[mr]$^2$ represents an index of the randomness of the polymer (F2). When this value is 2.0 or less, the spun-bonded nonwoven fabric is provided with sufficient elastic recovery, and the stickiness thereof is suppressed. The closer the value of [mm] $\times$ [rr]/[mr]$^2$ to 0.25, the higher the randomness.
From the viewpoint of obtaining sufficient elastic recovery as described above, the value of [mm] $\times$ [rr]/[mr]$^2$ is preferably more than 0.25 but 1.8 or less, more preferably from 0.5 to 1.5.

(1.1.2.6.2.5) Weight-Average Molecular Weight (Mw)

The polymer (F2) satisfies the following equation (e):

Equation (e): weight-average molecular weight (Mw) = from 10,000 to 200,000

When the polymer (F2), which is a propylene homopolymer, has a weight-average molecular weight of 10,000 or more, since the viscosity of the polymer (F2) is not excessively low and becomes rather moderate, fiber breakage during the production of the spun-bonded nonwoven fabric from the resin composition is suppressed. When the weight-average molecular weight is 200,000 or less, the viscosity of the polymer (F2) is not excessively high, so that the spinnability is improved.

The weight-average molecular weight of the polymer (F2) is preferably from 30,000 to 150,000, more preferably from 50,000 to 150,000. A method of measuring the weight-average-molecular weight of the polymer (F2) will be described below.

(1.1.2.6.2.6) Molecular Weight Distribution (Mw/Mn)

The polymer (F2) satisfies the following equation (f):

Equation (f): molecular weight distribution (Mw/Mn) < 4

When the polymer (F2) has a molecular weight distribution (Mw/Mn) of less than 4, the occurrence of stickiness in the resulting spun-bonded nonwoven fabric is suppressed. The molecular weight distribution of the polymer (F2) is preferably 3 or less.

Regarding the polymers (F1) and (F2), the weight-average molecular weight (Mw) is a weight-average molecular weight in terms of polystyrene, which is measured by a gel permeation chromatography (GPC) method using the below-described apparatus under the below-described conditions. The molecular weight distribution (Mw/Mn) is a value calculated from the number-average molecular weight (Mn) measured in the same manner, and the weight-average molecular weight (Mw).

[Measurement Conditions]

Column: TOSO GMHHR-H(S) HT
Detector: RI detector for liquid chromatogram WATERS 150C
Solvent: 1,2,4-trichlorobenzene
Measurement temperature: 145°C
Flow rate: 1.0 mL/min
Sample concentration: 2.2 mg/mL
Injection amount: 160 μL
Calibration curve: Universal Calibration
Analysis program: HT-GPC (Ver. 1.0)

(1.1.2.6.2.7) Melting Point (Tm-D)

The polymer (F2) preferably satisfies the following equation (g):

Equation (g): melting point (Tm-D) = from 0°C to 120°C

When the melting point (Tm-D) of the polymer (F2) is 0°C or higher, the occurrence of stickiness in the spun-bonded nonwoven fabric formed from the resin composition is suppressed, while when the melting point (Tm-D) is 120°C or lower, sufficient elastic recovery is obtained.

From this viewpoint, the melting point (Tm-D) is preferably from 0°C to 100°C, more preferably from 30°C to 100°C.

The melting point (Tm-D) of the polymer (F2) can be determined as the top of a peak observed on the highest temperature side of a melting endothermic curve obtained with the use of a differential scanning calorimeter (DSC-7, manufactured by PerkinElmer Co., Ltd.) by maintaining 10 mg of a sample in a nitrogen atmosphere at -10°C for 5 minutes and subsequently raising the temperature at a rate of 10°C/min.

(1.1.2.6.2.8) Melt Flow Rate

From the viewpoint of obtaining favorable spinnability and excellent stretching process suitability, the polymer (F2) has a melt flow rate of preferably from 1 g/10 min to 100 g/10 min, more preferably from 5 g/10 min to 100 g/10 min, still more preferably from 30 g/10 min to 70 g/10 min.

The melt flow rate of the polymer (F2) is measured by a method conforming to the ASTM Standard D-1238. As for the measurement conditions, the melt flow rate of the polymer (F2) is measured at 230°C with a load of 2.16 kg.

The polymer (F2) can be synthesized using a homogeneous catalyst that is a so-called metallocene catalyst, such as the one described in WO 2003/087172.

When the resin composition and the fibers contain at least one of the polymer (F1) or the polymer (F2), the content of the polymer (F1) and/or the polymer (F2) is preferably from 5% by mass to 30% by mass, more preferably from 10% by mass to 25% by mass, with respect to the total amount of the resin composition.

(1.1.2.7) Additive (G)

The resin composition may also contain an additive (G) in a range where the effects of the disclosure are exerted. Examples of the additive (G) include antioxidants, weathering stabilizers, light stabilizers, antistatic agents, slip agents, hydrophilic agents, antifogging agents, blocking inhibitors, lubricants, nucleating agents, dyes, pigments, natural oils, synthetic oils, waxes, and amide compounds. In the resin composition, these additives (G) may be used singly, or in combination of two or more kinds thereof.

The resin composition may contain an amide compound. The amide compound can function as a lubricant. Examples of the amide compound include fatty acid amides (e.g., fatty acid amides having from 15 to 22 carbon atoms). It is considered that a fatty acid amide having from 15 to 22 carbon atoms adsorbs to the fiber surfaces of the spun-bonded nonwoven fabric, and the fibers surfaces are thereby modified, as a result of which the flexibility, the texture, the blocking resistance, etc. are further improved, and adhesion of the fibers to various members such as rotating equipment in an apparatus used for an embossing process or the like is more effectively suppressed.

The term "number of carbon atoms of the fatty acid amide" used herein means the number of carbon atoms contained in a molecule, and the carbon atom constituting an amide bond is also included in the number of carbon atoms.

The number of carbon atoms of the fatty acid amide is preferably from 15 to 22, more preferably from 18 to 22. Examples of the fatty acid amide include fatty acid monoamide compounds, fatty acid diamide compounds, saturated fatty acid monoamide compounds, and unsaturated fatty acid diamide compounds. Specific examples of the fatty acid amide include palmitic acid amide (number of carbon atoms: 16), stearic acid amide (number of carbon atoms: 18), oleic acid amide (number of carbon atoms: 18), and erucic acid amide (number of carbon atoms: 22). In the resin composition, these amide compounds may be used singly, or in combination of two or more kinds thereof.

When the resin composition contains an amide compound, the content of the amide compound is preferably from 0.1% by mass to 5.0% by mass, more preferably from 0.1% by mass to 3.0% by mass, still more preferably from 0.1% by mass to 1.0% by mass, with respect to the total amount of the resin composition.

(1.1.3) Physical Properties of Spun-Bonded Nonwoven Fabric

In the spun-bonded nonwoven fabric, it is preferred that the air permeability is 280 $cm^3/cm^2 \cdot sec$ or more, and that a ratio of the strength index (N/25 mm) with respect to the basis weight ($g/m^2$) (hereinafter, also referred to as "ratio (strength index/basis weight)") is 0.70 $[(N/25\ mm)/(g/m^2)]$ or higher.

Methods of measuring each of the air permeability, the strength index, and the basis weight are the same as the methods described below in the section of Examples.

From the viewpoint of obtaining both satisfactory tensile strength and satisfactory air permeability in the spun-bonded nonwoven fabric, the ratio (strength index/basis weight) is more preferably from 0.70 $[(N/25\ mm)/(g/m^2)]$ to 2.0 $[(N/25\ mm)/(g/m^2)]$, still more preferably from 0.72 $[(N/25\ mm)/(g/m^2)]$ to 1.7 $[(N/25\ mm)/(g/m^2)]$, particularly preferably from 0.74 $[(N/25\ mm)/(g/m^2)]$ to 1.5 $[(N/25\ mm)/(g/m^2)]$.

From the viewpoint of obtaining both satisfactory tensile strength and satisfactory air permeability in the spun-bonded nonwoven fabric, the air permeability of the spun-bonded nonwoven fabric is preferably from 280 $cm^3/cm^2 \cdot sec$ to 1,000 $cm^3/cm^2 \cdot sec$, more preferably from 290 $cm^3/cm^2 \cdot sec$ to 800 $cm^3/cm^2 \cdot sec$, still more preferably from 300 $cm^3/cm^2 \cdot sec$ to 600 $cm^3/cm^2 \cdot sec$, particularly preferably from 310 $cm^3/cm^2 \cdot sec$ to 550 $cm^3/cm^2 \cdot sec$.

From the viewpoint of obtaining both satisfactory tensile strength and satisfactory air permeability in the spun-bonded nonwoven fabric, the strength index of the spun-bonded nonwoven fabric is preferably from 15 (N/25 mm) to 40 (N/25 mm), more preferably from 18 (N/25 mm) to 35 (N/25 mm), still more from preferably 20 (N/25 mm) to 30 (N/25 mm).

From the viewpoint of obtaining both satisfactory tensile strength and satisfactory air permeability in the spun-bonded nonwoven fabric, the basis weight of the spun-bonded nonwoven fabric is preferably from 1 g/m$^2$ to 100 g/m$^2$, more preferably from 5 g/m$^2$ to 80 g/m$^2$, still more preferably from 10 g/m$^2$ to 70 g/m$^2$.

(1.1.4) Configuration of Spun-Bonded Nonwoven Fabric

The spun-bonded nonwoven fabric may be a spun-bonded nonwoven fabric composed of a single layer containing sea-island fibers, a nonwoven fabric laminated body composed of two or more layers of spun-bonded nonwoven fabrics containing sea-island fibers, or a nonwoven fabric laminated body that includes a spun-bonded nonwoven fabric consisting of only sea-island fibers and a layer other than the spun-bonded nonwoven fabric. The layer other than the spun-bonded nonwoven fabric may be provided singly, or in two or more layers.

Examples of the layer other than the spun-bonded nonwoven fabric include knitted fabrics, woven fabrics, spun-bonded nonwoven fabrics other than a nonwoven fabric containing no sea-island fiber (hereinafter, also referred to as "other nonwoven fabrics"), and films. Examples of the other nonwoven fabrics include the above-described various nonwoven fabrics.

A method of forming a nonwoven fabric laminated body is not particularly limited, and examples thereof include: thermal fusion methods, such as hot embossing and ultrasonic fusion; mechanical entanglement methods, such as needle-punching and water-jetting; methods using an adhesive, such as a hot melt adhesive or a urethane adhesive; and various other methods, such as extrusion lamination.

It is preferred that the spun-bonded nonwoven fabric is partially press-bonded (preferably heat-fused).

Examples of a press-bonding method employed for partially press-bonding the spun-bonded nonwoven fabric include the use of a means such as ultrasonic waves, hot embossing using an embossing roll, and hot air-through.

The spun-bonded nonwoven fabric may include a press-bonded portion and a non-press-bonded portion. The press-bonded portion has an area ratio of preferably from 5% to 30%, more preferably from 5% to 20%, still more preferably from 8% to 19%. Thereby, the balance between the flexibility and the strength of the spun-bonded nonwoven fabric, etc. can be imprived.

The area ratio of the press-bonded portion is defined as a proportion of the area of the press-bonded portion with respect to an observed area of the spun-bonded nonwoven fabric, which is determined by collecting a test piece of 10 mm × 10 mm from the spun-bonded nonwoven fabric and observing the contact surface of the test piece with an embossing roll under an electron microscope (magnification rate: × 100). The area ratio of protruding parts formed on the embossing roll that can form the press-bonded portion is hereinafter also referred to as "embossed area ratio". A preferred range of the embossed area ratio is the same as the above-described preferred range of the area ratio of the press-bonded portion.

Examples of the shape of the press-bonded portion include a circular shape, an elliptical shape, an oval shape, a square shape, a rhombic shape, a rectangular shape, a quadrangular shape, and a continuous shape based on the aforementioned shapes.

(1.1.5) Use of Spun-Bonded Nonwoven Fabric

The air permeability of the spun-bonded nonwoven fabric is higher than that of other nonwoven fabrics having the same strength. Therefore, the spun-bonded nonwoven fabric can be preferably used in applications where performance such as high air permeability is demanded.

The use of the spun-bonded nonwoven fabric is not particularly limited, and the spun-bonded nonwoven fabric can be widely utilized in those applications where a spun-bonded nonwoven fabric is usually used. Examples of the use of the spun-bonded nonwoven fabric include filters, hygienic materials, medical members, packaging materials (e.g., deoxidants, hot packs, warm poultices, and food packaging materials), battery separators, lagging materials, heat insulating materials, protective suits, clothing members, electronic materials, and sound absorbing materials. Thereamong, the spun-bonded nonwoven fabric is preferably used as a hygienic material.

(1.2) Hygienic Material

The hygienic material according to a first aspect includes the spun-bonded nonwoven fabric according to the first aspect. Examples of the hygienic material include: absorbent articles, such as disposable diapers, disposable pants, sanitary napkins, urine absorbing pads, and pet sheets; medical sanitary materials, such as bandages, medical gauzes, towels, sheets, and poultices; industrial masks; and sanitary masks.

The hygienic material is not limited to the above, and can be preferably used in other hygienic material applications. The hygienic material may include a nonwoven fabric laminated body formed of two or more layers of the spun-bonded nonwoven fabrics according to the first aspect, or may include a nonwoven fabric laminated body that includes the spun-bonded nonwoven fabric according to the first aspect and a layer other than the nonwoven fabric according to the first aspect.

(1.3) Method of Producing Spun-Bonded Nonwoven Fabric

A method of producing a spun-bonded nonwoven fabric according to the first aspect includes melt-spinning a resin composition to form a continuous fiber group, and stretching the thus formed continuous fiber group with a cooling air. The resin composition contains a propylene polymer (A), a thermoplastic polyurethane-based elastomer (B), and a polymer (C) modified with at least one polar group selected from oxygen-containing groups, nitrogen-containing groups, sulfur-containing groups, or phosphorus-containing groups. The flow rate of the cooling air (hereinafter, also referred to as "stretching air flow rate") is 3,000 m/min or more.

In the method of producing a spun-bonded nonwoven fabric according to the first aspect, the continuous fiber group is introduced into a stretching section and stretched with the cooling air. The "stretching air flow rate" refers to a ratio of the flow rate ($Nm^3$/min) of the cooling air with respect to a cross-sectional area ($m^2$) of a portion of the stretching section that has the narrowest opening diameter (e.g., the below-described bottleneck portion 16a).

The method of producing a spun-bonded nonwoven fabric according to the first aspect has the above-described configuration; therefore, it can yield the spun-bonded nonwoven fabric according to the first aspect.

This effect is presumably attributed to, but not limited to, the following reason.

In the first aspect, the stretching air flow rate is 3,000 m/min or more. That is , strong stretching is applied to the continuous fiber group. It is presumed that a spun-bonded nonwoven fabric having a ratio (peak intensity/crystallinity) in the above-described range can be obtained.

Specifically, the spun-bonded nonwoven fabric according to the first aspect is produced using the resin composition as a raw material, for example, in the following manner. That is, the resin composition is introduced to and melted in an extruder. The resulting melt of the resin composition is spun using a spun-bonded nonwoven fabric forming machine equipped with plural spinnerets. The thus obtained continuous fiber group is stretched while controlling the air volume of a blower or the like. In this process, the continuous fiber group is cooled as required. Thereafter, the continuous fiber group is deposited on a collection surface of the spun-bonded nonwoven fabric forming machine to obtain a spun-bonded web. The thus obtained spun-bonded web is hot-pressed using an embossing roll, whereby the spun-bonded nonwoven fabric is obtained.

(1.3.1) One Example of Method of Producing Spun-Bonded Nonwoven Fabric

One example of a method of producing a spun-bonded nonwoven fabric according to the first aspect will now be described in detail referring to FIG. 1. In a closed-system spunbonding method, a continuous fiber group obtained by melt-spinning a resin composition is stretched while being cooled in a closed space.

A production apparatus 100 for a closed-system spunbonding method, which is illustrated in FIG. 1, is provided with a spinning section 10. The spinning section 10 includes an extruder 11, a spinneret 12, a cooling chamber 13, a cooling air supply unit 14, a cooling air supply unit 15, and a stretching section 16. The extruder 11 extrudes a resin composition. The spinneret 12 performs spinning of a melt of the resin composition. The cooling chamber 13 cools a continuous fiber group 1 spun from the spinneret 12. The cooling air supply unit 14 and the cooling air supply unit 15 each supply a cooling air A into the cooling chamber 13 and the stretching section 16. The stretching section 16 is where the continuous fiber group 1 is stretched.

First, a resin composition is introduced into the extruder 11. The resin composition introduced into the extruder 11 is melt-kneaded in the extruder 11. The resulting melt of the resin composition is extruded from the extruder 11.

The melt of the resin composition extruded from the extruder 11 is introduced into the spinneret 12. The melt of the resin composition introduced into the spinneret 12 is extruded from the spinneret 12 and thereby spun. As a result, the continuous fiber group 1 is formed.

The continuous fiber group 1 is introduced to the cooling chamber 13. The continuous fiber group 1 introduced to the cooling chamber 13 is cooled by the cooling air A. The cooling air A is supplied into the cooling chamber 13 and the stretching section 16 from at least one of the cooling air supply unit 14 or the cooling air supply unit 15. The cooled continuous fiber group 1 is introduced to the stretching section 16, which is arranged on the downstream side of the cooling chamber 13.

The stretching section 16 has a bottleneck portion 16a and a cylindrical portion 16b. The cylindrical portion 16b is formed on one end of the bottleneck portion 16a on the lower side (i.e., the side of a moving collection member 21) in the vertical direction (i.e., the direction of gravity). The bottleneck portion 16a is in the form of a narrow path. The cylindrical portion 16b is a cylindrical object. As illustrated in FIG. 1, the hollow part of the cylindrical portion 16b expands downward.

The continuous fiber group 1 introduced to the stretching section 16 is stretched by an increased speed of the cooling air in the bottleneck portion 16a. The continuous fiber group 1, thus stretched and passed through the cylindrical portion 16b, is dispersed and collected on the moving collection member 21.

The dispersed continuous fiber group 1 is efficiently collected on the moving collection member 21 by means of a suction unit 22. The suction unit 22 is arranged underneath the collection surface of the moving collection member 21. Then, a spun-bonded web 2 is formed.

Thereafter, the fibers contained in the spun-bonded web 2 are hot-pressed by, for example, an embossing roll (not illustrated), and bonded with each other, whereby a spun-bonded nonwoven fabric is obtained.

The melting temperature of the resin composition is melted is not particularly limited, if it is higher than the softening temperature or melting temperature of the resin composition, and is lower than the thermal decomposition temperature of the resin composition. It is set as appropriate in accordance with the physical properties, etc. of the resin composition.

The temperature of the spinneret 12 is appropriatly adjusted in accordance with the physical properties, etc. of the resin composition. Taking into consideration the physical properties of the propylene polymer (A) contained in the resin composition, the temperature of the spinneret 12 is preferably from 180°C to 240°C, more preferably from 190°C to 230°C, still more preferably from 200°C to 225°C.

A pore size of the spinneret 12 is not particularly limited, but, from the viewpoint of the extensibility of the resulting spun-bonded nonwoven fabric, it is preferably from 0.05 mm to 1.00 mm.

From the viewpoint of the extensibility of the resulting spun-bonded nonwoven fabric, the single-pore discharge amount of the melt of the resin composition from the spinneret 12 is preferably from 0.1 g/min to 3.0 g/min, more preferably from 0.3 g/min to 1.0 g/min.

The temperature of the cooling air A used for cooling the continuous fiber group extruded from the spinneret 12 is not particularly limited as long as it is a temperature at which the resin composition is solidified. The temperature of the cooling air A is preferably from 5°C to 50°C, more preferably from 10°C to 40°C, still more preferably from 15°C to 30°C.

A ratio ($m^2$/g) of the amount of the cooling air per a width of 1 m with respect to the single-pore discharge amount ($m^3$/min/m) is not particularly limited, and it is preferably from 1 $m^2$/g to 10,000 $m^2$/g, more preferably from 10 $m^2$/g to 1,000 $m^2$/g.

The stretching air flow rate is 3,000 m/min or more.

From the viewpoint of obtaining both satisfactory tensile strength and satisfactory air permeability in the resulting spun-bonded nonwoven fabric, the stretching air flow rate is preferably 3,200 m/min or more, more preferably 3,500 m/min or more, still more preferably 3,800 m/min or more.

The stretching air flow rate is not particularly limited and may be, for example, 6,000 m/min or less, 5,500 m/min or less.

The flow rate of the cooling air A supplied from each of the cooling air supply unit 14 and the cooling air supply unit 15, and the cross-sectional area of the bottleneck portion 16a are not particularly limited as long as the stretching air flow rate is in the above-described range, and may be selected as appropriate such that the stretching air flow rate is in a specific range.

In the method of producing a spun-bonded nonwoven fabric according to the first aspect, the fibers contained in the resulting spun-bonded nonwoven fabric may be partially heat-fused. The fibers contained in the spun-bonded nonwoven fabric may also be press-compacted using a nip roll prior to being heat-fused.

The method of producing a spun-bonded nonwoven fabric according to the first aspect has been described taking a closed-system spunbonding method as an example; however, the method of producing a spun-bonded nonwoven fabric according to the disclosure is not limited to a closed-system spunbonding method. The method of producing a spun-bonded nonwoven fabric according to the disclosure may be an open-system spunbonding method. In the open-system spunbonding method, a continuous fiber group obtained by melt-spinning the resin composition is cooled.

(2) Second Aspect

(2.1) Spun-Bonded Nonwoven Fabric

The spun-bonded nonwoven fabric according to a second aspect contains fibers formed of a resin composition. This resin composition contains: a propylene polymer (A); a crystal nucleating agent (b); and a polymer (C) modified with at least one polar group selected from oxygen-containing groups, nitrogen-containing groups, sulfur-containing groups, or phosphorus-containing groups. The fibers have a sea-island structure. A sea phase contained in the sea-island structure contains the propylene polymer (A). Island phases contained in the sea-island structure contain the crystal nucleating agent (b) A ratio of a peak intensity of an $\alpha$-crystals of the fibers with respect to a crystallinity of the fibers is 10.5 (intensity(counts)/%) or lower. The peak intensity of the $\alpha$-crystals represents a peak intensity of a (110) plane of the propylene polymer (A) at approximately $2\theta = 14°$ in an X-ray diffraction pattern measured by an X-ray diffractometer using

CuK$\alpha$ radiation.

The term "crystal nucleating agent" used herein refers to a substance that accelerates the crystallization of a thermoplastic resin in the field of thermoplastic resins.

The spun-bonded nonwoven fabric according to the second aspect has the above-described configuration; therefore, it exhibits an excellent tensile strength and a high air permeability. In other words, in the spun-bonded nonwoven fabric according to the second aspect, an effect of improving the air permeability while maintaining or improving the tensile strength, which properties are in a general trade-off relationship, is exerted.

(2.1.1) Fibers

The spun-bonded nonwoven fabric according to the second aspect contains fibers.

(2.1.1.1) Ratio (Peak Intensity/Crystallinity)

The ratio (peak intensity/crystallinity) is 10.5 (intensity(counts)/%) or lower. The ratio (peak intensity/crystallinity) is the same as that of the first aspect.

(2.1.1.2) Sea-Island Structure

In the second aspect, the fibers have a sea-island structure.

In the second aspect, the sea-island fibers are formed of a resin composition. This resin composition contains a propylene polymer (A), a crystal nucleating agent (b), and a polymer (C), and may further contain a polyethylene (D) if necessary.

The details of the resin composition will be described below.

The sea-island structure has a sea phase and plural island phases. The plural island phases exist (e.g., are dispersed) in the sea phase.

It is preferred that the sea phase contains the propylene polymer (A), and that the plural island phases each contain the crystal nucleating agent (b). When the resin composition further contains the polyethylene (D), it is preferred that the sea phase contains the propylene polymer (A), and that the plural island phases each contain an island phase of the crystal nucleating agent (b) and an island phase of the polyethylene (D).

The sea-island fibers have a fiber diameter of preferably 50 $\mu$m or less, more preferably 40 $\mu$m or less, still more preferably 30 $\mu$m or less, particularly preferably 28 $\mu$m or less. The smaller the fiber diameter, the superior the flexibility of the nonwoven fabric. From the viewpoint of the ease of handling, the fiber diameter of the sea-island fibers is preferably 10 $\mu$m or more. The sea-island fibers may be long fibers (staples) or short fibers (filaments). A cross-sectional shape of the sea-island fibers is not particularly limited, and examples thereof include a circular shape, an elliptical shape, and an irregular shape.

(2.1.1.2.1) Island Phases of Crystal Nucleating Agent (b)

When a cross-section of the fibers which is perpendicular to an axial direction is observed under a transmission electron microscope in a viewing area of 25.5 $\mu$m$^2$, it is preferred that island phases containing the crystal nucleating agent (b) have an average diameter of 0.095 $\mu$m or less, and that the number of the island phases containing the crystal nucleating agent (b) is 200 or more. Thereby, the spinnability can be improved, andsatisfactory tensile strength and satisfactory air permeability can be obtained in the spun-bonded nonwoven fabric.

A magnification rate of the transmission electron microscope is not particularly limited, and may be adjusted as appropriate such that the size and the number of the island phases can be measured at a cross-section of the fibers. For example, the magnification rate of the transmission electron microscope may be $\times$40,000.

Methods of measuring each of the average diameter and the number of the island phases containing the crystal nucleating agent (b) are the same as the methods described below in the section of Examples.

The average diameter of the island phases containing the crystal nucleating agent (b) is the same as exemplified above as the average diameter of the island phases containing the thermoplastic polyurethane-based elastomer (B) in the first aspect.

The number of the island phases containing the crystal nucleating agent (b) is the same as exemplified above as the number of the island phases containing the thermoplastic polyurethane-based elastomer (B) in the first aspect.

A ratio of the area of the island phases with respect to a total area of a cross-section of the sea-island fibers (hereinafter, referred to as "island phase area ratio") is preferably 1.0% or higher. When the island phase area ratio is from 1.0% or higher, oriented crystallization of the propylene polymer (A) is likely to be suppressed inside the sea-island fibers.

The island phase area ratio is preferably 20% or lower. When the island phase area ratio is 20% or lower, oriented crystallization of the propylene polymer (A) is likely to be suppressed inside the sea-island fibers. This allows the spun-bonded nonwoven fabric to have superior extensibility and superior spinnability.

A method of adjusting the island phase area ratio is not particularly limited, and examples thereof include a raw material adjustment method.

(2.1.1.2.2) Island Phases of Polyethylene (D)

In cases where the resin composition further contains the polyethylene (D), when a cross-section of the fibers which is perpendicular to an axial direction is observed under a transmission electron microscope in a viewing area of 25.5 $\mu m^2$, it is preferred that island phases containing the polyethylene (D) have an average diameter of 0.11 $\mu m$ or less, and that the number of the island phases containing the polyethylene (D) is 150 or more.

A magnification rate of the transmission electron microscope is not particularly limited, and may be adjusted as appropriate such that the size and the number of the island phases can be measured at a cross-section of the fibers. For example, the magnification rate of the transmission electron microscope may be $\times 40,000$.

The island phases of the polyethylene (D) are the same as exemplified above as the island phases of the polyethylene (D) in the first aspect.

(2.1.1.3) Forms, etc.

The form of the fibers contained in the spun-bonded nonwoven fabric is the same as exemplified above as the form of the fibers contained in the spun-bonded nonwoven fabric in the first aspect.

(2.1.2) Resin Composition

The fibers are formed of a resin composition.

The resin composition contains a propylene polymer (A), a thermoplastic polyurethane-based elastomer (B), and a polymer (C). The propylene polymer (A), the crystal nucleating agent (b), and the polymer (C) will each be described below in detail.

(2.1.2.1) Propylene Polymer (A)

The resin composition contains a propylene polymer (A).

The propylene polymer (A) is the same as exemplified above as the propylene polymer (A) in the first aspect.

(2.1.2.2) Crystal Nucleating Agent (b)

The resin composition contains a crystal nucleating agent (b).

As the crystal nucleating agent (b), any substance known as a crystal nucleating agent in the field of thermoplastic resins can be used, and examples thereof include organic crystal nucleating agents and inorganic crystal nucleating agents.

The term "organic crystal nucleating agent" refers to an organic compound that can form a three-dimensional network structure by self-assembly when heat-melted and then cooled, or melt-kneaded and thereby imparted with a shear fluidity. By adding such an organic crystal nucleating agent to a thermoplastic resin and subjecting the resultant to heat-melting and cooling, the growth of fine and uniform crystals of the thermoplastic resin is facilitated.

Specific examples of the organic crystal nucleating agents include the thermoplastic polyurethane-based elastomer (B), sorbitol compounds, phosphate compounds, amide compounds, and rosin compounds. The sorbitol compounds encompass aromatic ring-containing sorbitol compounds and aliphatic ring-containing sorbitol compounds, and preferred sorbitol compounds are aromatic ring-containing sorbitol compounds.

Specific examples of the inorganic crystal nucleating agents include talc, kaolinite, montmorillonite, synthetic mica, clay, zeolite, silica, graphite, carbon black, zinc oxide, magnesium oxide, titanium oxide, calcium sulfide, boron nitride, calcium carbonate, barium sulfate, aluminum oxide, neodymium oxide, and metal phenyl phosphonates. These inorganic crystal nucleating agents are preferably modified with an organic substance for the purpose of improving the dispersibility in the resin composition.

Particularly, the crystal nucleating agent (b) preferably contains the thermoplastic polyurethane-based elastomer (B). This allows the spun-bonded nonwoven fabric to have a superior tensile strength and a high air permeability.

The thermoplastic polyurethane-based elastomer (B) is the same as exemplified above as the thermoplastic polyurethane-based elastomer (B) in the first aspect.

From the viewpoint of obtaining both satisfactory tensile strength and satisfactory air permeability in the spun-bonded nonwoven fabric, the content of the crystal nucleating agent (b) is preferably from 2.0% by mass to 20.0% by mass, more preferably from 2.5% by mass to 19.0% by mass, still more preferably from 3.0% by mass to 18.0% by mass, with respect to a total amount of the resin composition.

A case where the crystal nucleating agent (b) is the thermoplastic polyurethane-based elastomer (B) in the second aspect will now be described.

(2.1.2.3) Polymer (C)

The resin composition contains a polymer (C).

The polymer (C) is the same as exemplified above as the polymer (C) in the first aspect.

(2.1.2.4) Polyethylene (D)

The resin composition may also contain a polyethylene (D).

The polyethylene (D) is the same as exemplified above as the polyethylene (D) in the first aspect.

(2.1.2.5) Low-Molecular-Weight Olefin Polymer (E)

The resin composition may also contain a low-molecular-weight olefin polymer (E).

The low-molecular-weight olefin polymer (E) is the same as exemplified above as the low-molecular-weight olefin polymer (E) in the first aspect.

(2.1.2.6) Other Polymer (F)

From the viewpoint of improving the extensibility of the fibers, the resin composition may also contain other polymer (F).

The other polymer (F) is the same as exemplified above as the other polymer (F) in the first aspect.

(2.1.2.7) Additive (G)

The resin composition may also contain an additive (G) in a range where the effects of the disclosure are exerted.

The additive (G) is the same as exemplified above as the additive (G) in the first aspect.

(2.1.3) Physical Properties of Spun-Bonded Nonwoven Fabric

In the spun-bonded nonwoven fabric, it is preferred that the air permeability is 280 $cm^3/cm^2$·sec or more, and that a ratio of the strength index (N/25 mm) with respect to the basis weight ($g/m^2$) (hereinafter, also referred to as "ratio (strength index/basis weight)") is 0.70 [(N/25 mm)/($g/m^2$)] or higher.

Methods of measuring each of the air permeability, the strength index, and the basis weight are the same as the methods described below in the section of Examples.

The ratio (strength index/basis weight) is the same as exemplified above as the ratio (strength index/basis weight) in the first aspect.

The air permeability of the spun-bonded nonwoven fabric is the same as exemplified above as the air permeability in the first aspect.

The strength index of the spun-bonded nonwoven fabric is the same as exemplified above as the strength index in the first aspect.

The basis weight of the spun-bonded nonwoven fabric is the same as exemplified above as the basis weight in the first aspect.

(2.1.4) Configuration of Spun-Bonded Nonwoven Fabric

The configuration of the spun-bonded nonwoven fabric is the same as exemplified above as the configuration of the spun-bonded nonwoven fabric in the first aspect.

(2.1.5) Use of Spun-Bonded Nonwoven Fabric

The use of the spun-bonded nonwoven fabric is the same as exemplified above as the use of the spun-bonded nonwoven fabric in the first aspect.

(2.2) Hygienic Material

The hygienic material according to the second aspect includes the spun-bonded nonwoven fabric according to the second aspect. The hygienic material according to the second aspect is the same as the hygienic material according to the first aspect, except that the hygienic material according to the second aspect includes the spun-bonded nonwoven fabric according to the second aspect in place of the spun-bonded nonwoven fabric according to the first aspect.

(2.3) Method of Producing Spun-Bonded Nonwoven Fabric Layer

A method of producing a spun-bonded nonwoven fabric according to the second aspect includes melt-spinning a resin composition to form a continuous fiber group, and stretching the thus formed continuous fiber group with a cooling air. The resin composition contains a propylene polymer (A), a crystal nucleating agent (b), and a polymer (C) modified with at least one polar group selected from oxygen-containing groups, nitrogen-containing groups, sulfur-containing groups, or phosphorus-containing groups. The flow rate of the cooling air (hereinafter, also referred to as "stretching air flow rate") is 3,000 m/min or more.

The method of producing a spun-bonded nonwoven fabric according to the second aspect has the above-described configuration; therefore, it can yield the spun-bonded nonwoven fabric according to the second aspect.

The method of producing a spun-bonded nonwoven fabric according to the second aspect is the same as the method of producing a spun-bonded nonwoven fabric according to the first aspect, except that the resin composition contains the crystal nucleating agent (b) in place of the thermoplastic polyurethane-based elastomer (B).

EXAMPLES

The disclosure will now be described more concretely by way of Examples; however, the disclosure is not limited to the below-described Examples. Those materials, amounts of use, ratios, treatment procedures, etc. that are indicated in the below-described Examples can be modified as appropriate without departing from the spirit of the present invention.

[1] Measurement Methods and Evaluation Methods

The physical property values, etc. in Examples and Comparative Examples were measured and evaluated by the following methods.

[1.1] Spinnability

From the spinning results of the spun-bonded nonwoven fabrics shown in Examples, the spinnability was evaluated based on the following criteria. An acceptable evaluation of the spinnability is "A".

A: Spinning was performed with no problem, and a spun-bonded nonwoven fabric was produced.
B: Spinning itself was performed; however, a spun-bonded nonwoven fabric could not be produced due to the breakage of a large number of fibers.

[1.2] Confirmation of Sea-Island Structure, Number of Island Phases, Average Diameter of Island Phases, and Area Ratio of Island Phases

A fiber was removed from each spun-bonded nonwoven fabric and embedded in paraffin. The thus obtained measurement sample was placed on a microtome such that its blade was parallel to the direction perpendicular to the axial direction of the fiber, and a cross-section of the fiber was obtained by slicing the measurement sample along the direction perpendicular to the axial direction of the fiber and then stained with $RuO_4$, after which this measurement sample was sliced again to prepare an ultrathin section. The thus prepared ultrathin section was reinforced with carbon, and a stained cross-section of this ultrathin section was observed under a transmission electron microscope (TEM). A sea-island structure was observed at the cross-section of the fiber. An island phase that was stained with $RuO_4$ in black either entirely or in an outer peripheral part was defined as an island phase containing a thermoplastic polyurethane-based elastomer (B), while a gray-white island phase that was not stained with $RuO_4$ was defined as an island phase containing a polyethylene (D).

As the transmission electron microscope, a transmission electron microscope Model H-7650 manufactured by Hitachi High-Tech Science Corporation was used, and the observation magnification rate was set at ×40,000.

The diameters of island phases were determined by image analysis using an image processing software WinROOF2018 manufactured by Mitani Corporation. Specifically, in a captured image, a rectangular region having an area of 25.5 $\mu$m2 (4.25 $\mu$m in length and 6.00 $\mu$m in width) was defined as an analysis area (viewing area), and island phases containing the thermoplastic polyurethane-based elastomer (B) and island phases containing the polyethylene (D) were binarized, after which the number of each kind of island phase was counted, and the equivalent circle diameter was calculated. An arithmetic mean of the equivalent circle diameter of island phases, which was calculated based on the number and the equivalent circle diameters of the island phases containing the thermoplastic polyurethane-based elastomer (B), was defined as the average diameter of the island phases containing the thermoplastic polyurethane-based elastomer (B). Further, an arithmetic mean of the equivalent circle diameter of island phases, which was calculated based on the number and the equivalent circle diameters of the island phases containing the polyethylene (D), was defined as the average diameter of the island phases containing the polyethylene (D).

An area ratio (B) of the island phases containing the thermoplastic polyurethane-based elastomer (B) was defined as a value obtained by dividing a total area of the island phases containing the thermoplastic polyurethane-based elastomer (B) by the analysis area of 25.5 $\mu$m2.

[1.3] Peak Intensity of $\alpha$-Crystals, Crystallinity, and Ratio (Peak Intensity/Crystallinity)

Using a wide-angle X-ray diffractometer (EMPYREAN manufactured by Spectris Co., Ltd., X-ray source: CuKa, output: 45 kV, 40 mA, detector: PIXcel3D), fibers cut out from each spun-bonded nonwoven fabric excluding embossed parts were loaded to a sample holder, and a wide-angle X-ray diffraction profile was measured while rotating the holder. From the results of a diffraction profile obtained by this measurement of wide-angle X-ray diffraction profile, the peak area derived from crystalline parts and the peak area derived from amorphous parts were determined, and the crystallinity was calculated by the following equation:

Crystallinity [%] = Peak area derived from crystalline parts/(Peak area derived from crystalline parts + Halo area derived from amorphous parts) $\times$ 100

From the results of the diffraction profile, the maximum peak intensity (peak intensity of $\alpha$-crystals) representing the (110) plane of a propylene polymer (A) at approximately $2\theta$ = 14° was determined.
A ratio of the peak intensity of $\alpha$-crystals with respect to the crystallinity (peak intensity/crystallinity) was calculated.

[1.4] Average Fiber Diameter

Using an electron microscope ("S-3500N" manufactured by Hitachi, Ltd.), an image of fibers of each spun-bonded nonwoven fabric was captured at a magnification rate of $\times$1,000. In the thus obtained image, the diameter of a fiber having a measurable diameter was measured, and the image-capturing and the measurement were repeated until the total number of measured fibers exceeded 100. An arithmetic mean of the thus measured fiber diameters was defined as the average fiber diameter ($\mu$m).

[1.5] Basis Weight

From each spun-bonded nonwoven fabric, ten test pieces of 250 mm (machine direction: MD) $\times$ 200 mm (cross direction: CD) were collected. These test pieces were collected at ten arbitrary spots in both the MD and the CD of the spun-bonded nonwoven fabric. Subsequently, the mass (g) of each of the thus collected test pieces was measured using an electronic even balance (manufactured by Kensei Kogyo Co., Ltd.), and an average mass of the test pieces was calculated. The thus calculated average value was converted into the mass (g) per 1 m2 and rounded off to the nearest whole number, and the resulting value was defined as the basis weight [g/m2].

[1.6] Tensile Strength, Strength Index, and Ratio (Strength Index/Basis Weight)

Measurement was performed for each spun-bonded nonwoven fabric in accordance with JIS L1906. A test piece of 25 mm in width $\times$ 200 mm in length was collected from the spun-bonded nonwoven fabric, and the tensile strength was measured at five spots in the MD and five spots in the CD using a tensile tester at a chuck distance of 100 mm and a head speed of 100 mm/min. The average value of the thus measured tensile strength in each direction was calculated to determine the MD tensile strength (N/25 mm) and the CD tensile strength (N/25 mm).
The strength index and the ratio (strength index/basis weight) were calculated using the following equations (X1) and (X2).

Strength index = $(((MD\ tensile\ strength)^2 + (CD\ tensile\ strength)^2)/2)^{0.5}$ Equation (X1):

## Equation (X2): Ratio (strength index/basis weight) = Strength index/Basis weight

An acceptable range of the ratio (strength index/basis weight) is 0.7 [(N/25 mm)/(g/m$^2$)] or higher.

[1.7] MD Maximum Elongation [%] and CD Maximum Elongation [%]

In accordance with JIS L1906 - 6.12.1 [Method A], five test pieces of 25 cm in the machine direction (MD) $\times$ 5 cm in the cross direction (CD) were collected from each spun-bonded nonwoven fabric in a thermostatic chamber having a temperature of 20 $\pm$ 2°C and a humidity of 65 $\pm$ 2% as prescribed in JIS Z8703 (Standard Atmospheric Conditions for Testing). For each of these five test pieces, a tensile test was conducted using a tensile tester (INSTRON 5564, manufactured by Instron Japan Co., Ltd.) at a chuck distance of 100 mm and a tensile speed of 300 mm/min to measure the tensile load, and the elongation at a strength giving the average of the thus measured maximum tensile load values was defined as the maximum elongation [%] and used as an index for evaluating the extensibility. Specifically, the MD maximum elongation [%] is calculated by the following equation (X3), while the CD maximum elongation [%] is calculated by the following equation (X4).

Maximum elongation [%] = {(First maximum length - 25 cm)/25 cm} $\times$ 100 Equation (X3):

Maximum elongation [%] = {(Second maximum length - 5 cm)/5 cm} $\times$ 100 Equation (X4):

In Equation (X3), the "first maximum length" represents the length [cm] of a test piece exhibiting a tensile strength in the MD. In Equation (X4), the "second maximum length" represents the length [cm] of the test piece exhibiting the tensile strength in the CD.
An acceptable range of the MD maximum elongation is 190% or more, and an acceptable range of the CD maximum elongation is 150% or more.

[1.8] Air Permeability

For a total of five spots in the center and four corners of each spun-bonded nonwoven fabric (100 mm $\times$ 100 mm), the air permeation amount was measured in accordance with JIS L1096 at a pressure difference of 125 Pa using a Frazier-type tester, and the average of the thus measured values was defined as the air permeability.
An acceptable range of the air permeability is 280 [cm$^3$/cm$^2$·sec] or more.

[1.9] Uniformity Index

Each spun-bonded nonwoven fabric was measured using a formation tester "FMT-MIII) manufactured by Nomura Shoji Co., Ltd., and the average of the thus measured uniformity index values (n = 5) was defined as the uniformity index. A smaller uniformity index indicates a superior uniformity.
The uniformity index is represented by $10\sigma/E$, wherein $\sigma$ represents the standard deviation of the density unevenness of the spun-bonded nonwoven fabric, and E is obtained by measuring the light transmittance (T) of the spun-bonded nonwoven fabric. E is represented by E = 2 - logT and, when the transmittance is close to 100% (poor uniformity), E $\approx$ 0 and the uniformity index shows an infinitely large number.

[1.9] Theoretical Resin Density

The theoretical resin density was calculated by the following equation (X5):

Theoretical resin density (g/cm$^3$) = 1/($\Sigma$(Composition ratio (-)/Density (g/cm$^3$))) Equation (X5):

$\Sigma$(Composition ratio (-)/Density (g/cm$^3$)) = Content ratio (% by mass) of propylene homopolymer (A-1) with respect to total amount of resin composition/Density (g/cm$^3$) of propylene homopolymer (A-1) + Content ratio (% by mass) of thermoplastic polyurethane-based elastomer (B-1) with respect to total amount of resin composition/Density (g/cm$^3$) of

thermoplastic polyurethane-based elastomer (B-1) + Content ratio (% by mass) of polymer (C-1) with respect to total amount of resin composition/Density (g/cm$^3$) of polymer (C-1) + Content ratio (% by mass) of polymer (C-2) with respect to total amount of resin composition/Density (g/cm$^3$) of polymer (C-2)

It is noted here that the density of the below-described propylene homopolymer (A-1) was set at 0.91 g/cm$^3$, the density of the below-described TPU (B-1) was set at 1.20 g/cm$^3$, the density of the below-described polymer (C-1) was set at 0.91 g/cm$^3$, the density of the below-described polymer (C-2) was set at 0.95 g/cm$^3$, and the density of the below-described polyethylene (D-1) was set at 0.95 g/cm$^3$.

[1.10] Thickness

Ten test pieces of 100 mm (machine direction: MD) × 100 mm (cross direction: CD) were collected from each spun-bonded nonwoven fabric, the center thickness of each test piece was measured, and an average value of the ten test pieces was calculated. The thus calculated average value was defined as the thickness of the spun-bonded nonwoven fabric. For the measurement of the thickness, a thickness gauge having a load of 125 gf/cm$^2$ (probe diameter: 5 mmφ) was used.

[1.11] Fiber Filling Rate and Porosity

The fiber filling rate (corresponding to the amount of fibers per unit volume) of each spun-bonded nonwoven fabric is represented by the following equation (X6). The porosity (voids per unit volume) of the spun-bonded nonwoven fabric is represented by the following equation (X7).

Fiber filling rate (%) = (Basis weight (g/m$^2$)/Thickness (mm)/Theoretical resin density (g/cm$^3$)/1,000) × 100          Equation (X6):

$$\text{Equation (X7): Porosity (\%) = 100 - Fibers filling rate (\%)}$$

[2] Preparation

[2.1] Preparation of Propylene Polymer (A)

As a propylene polymer (A), a propylene homopolymer (A-1) having an MFR (measured in accordance with ASTM D1238 at a temperature of 230°C and a load of 2.16 kg) of 60 g/10 min, a density of 0.91 g/cm$^3$, and a melting point (Tm) of 160°C was prepared.

[2.2] Production of Thermoplastic Polyurethane-Based Elastomer (B)

As a thermoplastic polyurethane-based elastomer (B), TPU (B-1) was produced in the following manner. A polyester polyol having a number-average molecular weight of 1,932: 71.7 parts by mass, 1,4-butanediol (hereinafter, also referred to as "BD"): 4.8 parts by mass, pentaerythritol tetrakis[3-(3,5-di-t-butyl-4-hydroxyphenyl)propionate] (hereinafter, simply referred to as "antioxidant-1"): 0.3 parts by mass, and a polycarbodiimide: 0.3 parts by mass were mixed, and 22.9 parts by mass of 4,4'-diphenylmethane diisocyanate (hereinafter, also referred to as "MDI") was added to the resultant, which was subsequently thoroughly mixed with high-speed stirring and then allowed to react at 160°C for 1 hour.

The thus obtained reaction product was pulverized, and 100 parts by mass of the resulting pulverized product was mixed with 0.8 parts by mass of ethylene-bis stearic acid amide, 0.5 parts by mass of triethylene glycol bis[3-(3,5-di-t-butyl-4-hydroxyphenyl)propionate] (hereinafter, also referred to as "antioxidant-2"), and 0.8 parts by mass of ethylene-bis oleic acid amide (hereinafter, also referred to as "EOA"), after which the resultant was melt-kneaded and granulated using an extruder (preset temperature: 210°C) to obtain TPU (B-1).

The thus obtained TPU (B-1) had the following physical properties: shore A hardness = 82, melting point (Tm) (high-melting-point side) = 162.2°C, heat of fusion = 11.4 mJ/mg, solidification start temperature = 155°C, ratio of heat of fusion of hard domain = 0.58, and melt viscosity = 1,100 Pa·s.

[2.3] Preparation of Polymers (C)

As a polymer (C-1), TUFTEC "Grade MP10" manufactured by Asahi Kasei Corporation (type: amine-modified hydrogenated styrene-based thermoplastic elastomer, polar group: amino groups, main chain: hydrogenated styrene-based block copolymer) was used.

As a polymer (C-2), UMEX "Grade 1001" manufactured by Sanyo Chemical Industries, Inc. (type: carboxylic acid anhydride-modified polypropylene-based resin, polar group: carboxylic acid anhydride groups, main chain: polyolefin) was used.

[2.4] Preparation of Polymer (I)

As a polymer (I), TUFTEC "Grade H1053" manufactured by Asahi Kasei Corporation (type: hydrogenated styrene-based thermoplastic elastomer, polar group: not detected, main chain: hydrogenated styrene-based block copolymer) was used.

[2.5] Preparation of Polyethylene (D)

As a polyethylene (D-1), a polyethylene having an MFR (measured in accordance with ASTM D1238 at a temperature of 190°C and a load of 2.16 kg) of 5 g/10 min, a density of 0.95 g/cm$^3$, and a melting point (Tm) of 134°C was used.

[3] Examples and Comparative Examples

[3.1] Example 1

Using the production apparatus illustrated in FIG. 1, a spun-bonded nonwoven fabric was produced in the following manner. A mixture of the following materials was melted using a 75-mmφ extruder: 80.6% by mass of the propylene homopolymer (A-1) having an MFR (measured in accordance with ASTM D1238 at a temperature of 230°C and a load of 2.16 kg) of 60 g/10 min, a density of 0.910 g/cm$^3$, and a melting point (Tm) of 160°C, 16.1% by mass of the TPU (B-1), and 3.2% by mass of the polymer (C-1). The thus molten mixture was spun using the spun-bonded nonwoven fabric production apparatus equipped with a 1,093-hole spinneret having a pore size of 0.6 mm under the conditions in which the resin temperature and the die temperature were both 205°C and the cooling air temperature was 20°C (single-pore discharge amount of the mixture: 0.60 g/min, stretching air flow rate: 4,190 m/min), and the resultant was deposited on a collection belt and then hot-pressed using an embossing roll (embossed area ratio (hot-press rate)) = 18%, embossing temperature = 120°C), whereby a spun-bonded nonwoven fabric having a total basis weight of 30 g/m$^2$ was produced. The "stretching air flow rate" refers to a flow rate of cooling air (Nm$^3$/min) with respect to a cross-sectional area (m$^2$) of the bottleneck portion of the stretching section.

[3.2] Comparative Example 1

A spun-bonded nonwoven fabric having a total basis weight of 30 g/m$^2$ was produced by performing the same operations as in Example 1, except that the stretching air flow rate was changed from 4,190 m/min to 2,841 m/min.

[3.3] Comparative Example 2

The same operations were performed as in Example 1, except that 83.3% by mass of the propylene homopolymer (A-1) having an MFR (measured in accordance with ASTM D1238 at a temperature of 230°C and a load of 2.16 kg) of 60 g/10 min, a density of 0.910 g/cm$^3$, and a melting point (Tm) of 160°C and 16.7% by mass of the TPU (B-1) were melted using a 75-mmcp extruder. However, a spun-bonded nonwoven fabric could not be produced due to the breakage of a large number of fibers in the spinning stage.

Therefore, a sample was prepared in the following manner for transmission electron microscopy and wide-angle X-ray diffraction measurement. Fibers of the above-described mixture were deposited on an arbitrary fabric such that the amount of the fibers was 30 g/m$^2$, and the fibers were hot-pressed along with the fabric using an embossing roll (embossed area ratio (hot-press rate)) = 18%, embossing temperature = 120°C), after which a spun-bonded nonwoven fabric was peeled off from the resulting embossed laminated body and used as a measurement sample.

[3.4] Comparative Example 3

A spun-bonded nonwoven fabric having a total basis weight of 30 g/m$^2$ was produced by performing the same operations as in Comparative Example 2, except that the stretching air flow rate was changed from 4,190 m/min to 2,841 m/min.

[3.5] Comparative Example 4

A spun-bonded nonwoven fabric having a total basis weight of 30 g/m² was produced by performing the same operations as in Example 1, except that a mixture of 96.2% by mass of the propylene homopolymer (A-1) having an MFR (measured in accordance with ASTM D1238 at a temperature of 230°C and a load of 2.16 kg) of 60 g/10 min, a density of 0.910 g/cm³, and a melting point (Tm) of 160°C, and 3.8% by mass of the polymer (C-1) was melted using a 75-mmφ extruder.

[3.6] Comparative Example 5

A spun-bonded nonwoven fabric having a total basis weight of 30 g/m² was produced by performing the same operations as in Example 1, except that 100.0% by mass of the propylene homopolymer (A-1) having an MFR (measured in accordance with ASTM D1238 at a temperature of 230°C and a load of 2.16 kg) of 60 g/10 min, a density of 0.910 g/cm³, and a melting point (Tm) of 160°C was melted using a 75-mmφ extruder.

[3.7] Example 2

A spun-bonded nonwoven fabric having a total basis weight of 30 g/m² was produced by performing the same operations as in Example 1, except that a mixture of 75.8% by mass of the propylene homopolymer (A-1) having an MFR (measured in accordance with ASTM D1238 at a temperature of 230°C and a load of 2.16 kg) of 60 g/10 min, a density of 0.910 g/cm³, and a melting point (Tm) of 160°C, 16.1 % by mass of the TPU (B-1), 3.2% by mass of the polymer (C-1), and 4.8% by mass of the polyethylene (D-1) was melted using a 75-mmφ extruder.

[3.8] Example 3

A spun-bonded nonwoven fabric having a total basis weight of 30 g/m² was produced by performing the same operations as in Example 2, except that the polymer (C-1) was changed to the polymer (C-2).

[3.9] Comparative Example 6

The same operations were performed as in Example 2, except that the polymer (C-1) was changed to the polymer (I). However, a spun-bonded nonwoven fabric could not be produced due to the breakage of a large number of fibers in the spinning stage. Therefore, a sample for transmission electron microscopy and wide-angle X-ray diffraction measurement was prepared in the manner described in Comparative Example 2.

[3.10] Comparative Example 7

A spun-bonded nonwoven fabric having a total basis weight of 30 g/m² was produced by performing the same operations as in Comparative Example 6, except that the stretching air flow rate in Comparative Example 5 was changed from 4,190 m/min to 2,841 m/min.

[3.11] Comparative Example 8

The same operations were performed as in Example 1, except that a mixture of 78.3% by mass of the propylene homopolymer (A-1) having an MFR (measured in accordance with ASTM D1238 at a temperature of 230°C and a load of 2.16 kg) of 60 g/10 min, a density of 0.910 g/cm³, and a melting point (Tm) of 160°C, 16.7% by mass of the TPU (B-1), and 5.0% by mass of the polyethylene (D-1) was melted using a 75-mmφ extruder. However, a spun-bonded nonwoven fabric could not be produced due to the breakage of a large number of fibers in the spinning stage. Therefore, a sample for transmission electron microscopy and wide-angle X-ray diffraction measurement was prepared in the manner described in Comparative Example 2.

[3.12] Comparative Example 9

A spun-bonded nonwoven fabric having a total basis weight of 30 g/m² was produced by performing the same operations as in Comparative Example 8, except that the stretching air flow rate in Comparative Example 6 was changed from 4,190 m/min to 2,841 m/min.

[3.13] Comparative Example 10

A spun-bonded nonwoven fabric having a total basis weight of 30 g/m$^2$ was produced by performing the same operations as in Example 1, except that a mixture of 90.4% by mass of the propylene homopolymer (A-1) having an MFR (measured in accordance with ASTM D1238 at a temperature of 230°C and a load of 2.16 kg) of 60 g/10 min, a density of 0.910 g/cm$^3$, and a melting point (Tm) of 160°C, 3.8% by mass of the polymer (C-1), and 5.8% by mass of the polyethylene (D-1) was melted using a 75-mm$\varphi$ extruder.

[3.14] Comparative Example 11

A spun-bonded nonwoven fabric having a total basis weight of 30 g/m$^2$ was produced by performing the same operations as in Example 1, except that a mixture of 94.0% by mass of the propylene homopolymer (A-1) having an MFR (measured in accordance with ASTM D1238 at a temperature of 230°C and a load of 2.16 kg) of 60 g/10 min, a density of 0.910 g/cm$^3$, and a melting point (Tm) of 160°C and 6.0% by mass of the polyethylene (D-1) was melted using a 75-mm$\varphi$ extruder.

[3.15] Example 4

A spun-bonded nonwoven fabric having a total basis weight of 29 g/m$^2$ was produced by performing the same operations as in Example 1, except that a mixture of 86.2% by mass of the propylene homopolymer (A-1) having an MFR (measured in accordance with ASTM D1238 at a temperature of 230°C and a load of 2.16 kg) of 60 g/10 min, a density of 0.910 g/cm$^3$, and a melting point (Tm) of 160°C, 4.6% by mass of the TPU (B-1), 3.7% by mass of the polymer (C-1), and 5.5% by mass of the polyethylene (D-1) was melted using a 75-mm$\varphi$ extruder.

[3.16] Comparative Example 12

A spun-bonded nonwoven fabric having a total basis weight of 30 g/m$^2$ was produced by performing the same operations as in Example 1, except that a mixture of 89.5% by mass of the propylene homopolymer (A-1) having an MFR (measured in accordance with ASTM D1238 at a temperature of 230°C and a load of 2.16 kg) of 60 g/10 min, a density of 0.910 g/cm$^3$, and a melting point (Tm) of 160°C, 4.8% by mass of the TPU (B-1), and 5.7% by mass of the polyethylene (D-1) was melted using a 75-mm$\varphi$ extruder.

[Table 1]

| | | | | Example 1 | Comparative Example 1 | Comparative Example 2 | Comparative Example 3 | Comparative Example 4 | Comparative Example 5 | Example 2 | Example 3 |
|---|---|---|---|---|---|---|---|---|---|---|---|
| Components (% by mass) | Propylene polymer (A) | | | 80.6 | 80.6 | 83.3 | 83.3 | 96.2 | 100.0 | 75.8 | 75.8 |
| | Thermoplastic polyurethane-based elastomer (B) | | | 16.1 | 16.1 | 16.7 | 16.7 | - | - | 16.1 | 16.1 |
| | Polymer (C) | (C-1) | | 3.2 | 3.2 | - | - | 3.8 | - | 3.2 | - |
| | | (C-2) | | - | - | - | - | - | - | - | 3.2 |
| | | (C-3) | | - | - | - | - | - | - | - | |
| | Polyethylene (D) | | | - | - | - | - | - | - | 4.8 | 4.8 |
| Spinning condition | Stretching air flow rate | | [m/min] | 4,190 | 2,841 | 4,190 | 2,841 | 4,190 | 4,190 | 4,190 | 4,190 |
| Fiber properties | Spinnability | | | A | A | B | A | A | A | A | A |
| | (B) | Number of islands | [count] | 611 | 620 | 242 | 229 | - | - | 638 | 528 |
| | | Average diameter of islands | [μm] | 0.086 | 0.083 | 0.139 | 0.131 | - | - | 0.081 | 0.092 |
| | | Area ratio of islands | [%] | 16.6 | 17.0 | 17.1 | 17.6 | - | - | 17.3 | 17.3 |
| | (D) | Number of islands | [count] | - | - | - | - | - | - | 256 | 223 |
| | | Average diameter of islands | [μm] | - | - | - | - | - | - | 0.077 | 0.081 |
| | (110) plane peak intensity | | [int[counts]] | 543 | 582 | 581 | 563 | 629 | 622 | 522 | 530 |
| | Crystallinity | | [%] | 53 | 52 | 54 | 51 | 55 | 56 | 55 | 54 |
| | Peak intensity/crystallinity | | [int[counts]/%] | 10.2 | 11.2 | 10.8 | 11.0 | 11.4 | 11.1 | 9.5 | 9.8 |
| | Fiber diameter | | [μm] | 24 | 30 | | 28 | 23 | 23 | 25 | 25 |
| | Basis weight | | [g/m$^2$] | 30 | 30 | | 30 | 30 | 30 | 30 | 30 |

(continued)

| | | | | Example 1 | Comparative Example 1 | Comparative Example 2 | Comparative Example 3 | Comparative Example 4 | Comparative Example 5 | Example 2 | Example 3 |
|---|---|---|---|---|---|---|---|---|---|---|---|
| Evaluation results | Nonwoven fabric properties | MD maximum strength | [N/25mm] | 31 | 14 | Non-woven fabric could not be pro-duced | 15 | 27 | 28 | 30 | 30 |
| | | CD maximum strength | [N/25mm] | 11 | 11 | | 10 | 10 | 10 | 11 | 12 |
| | | Strength index | [N/25mm] | 23 | 13 | | 13 | 21 | 21 | 23 | 23 |
| | | Strength index/Basis weight | [N/25mm/g/m$^2$] | 0.77 | 0.42 | | 0.43 | 0.69 | 0.71 | 0.76 | 0.77 |
| | | MD elongation | [%] | 203 | 155 | | 161 | 186 | 193 | 221 | 211 |
| | | CD elongation | [%] | 154 | 130 | | 123 | 147 | 152 | 162 | 156 |
| | | Air permeability | [cm$^3$/cm$^2$·s] | 302 | 520 | | 511 | 256 | 267 | 337 | 312 |
| | | Uniformity index | [-] | 353 | 430 | | 428 | 361 | 360 | 349 | 353 |
| | | Theoretical resin den-sity | [g/cm$^3$] | 0.95 | 0.95 | | 0.95 | 0.91 | 0.91 | 0.95 | 0.95 |
| | | Thickness | [mm] | 0.14 | 0.12 | | 0.12 | 0.12 | 0.12 | 0.16 | 0.15 |
| | | Filling rate | [%] | 23 | 26 | | 26 | 27 | 27 | 19 | 21 |
| | | Porosity | [%] | 77 | 74 | | 74 | 73 | 73 | 81 | 79 |

[Table 2]

| Components (% by mass) | | | Compara-tive Example 6 | Compara-tive Example 7 | Compara-tive Example 8 | Compara-tive Example 9 | Compara-tive Example 10 | Compara-tive Example 11 | Exam-ple 4 | Compara-tive Example 12 |
|---|---|---|---|---|---|---|---|---|---|---|
| Components (% by mass) | Propylene polymer (A) | | 75.8 | 75.8 | 78.3 | 78.3 | 90.4 | 94.0 | 86.2 | 89.5 |
| | Thermoplastic polyurethane-based elastomer (B) | | 16.1 | 16.1 | 16.7 | 16.7 | - | - | 4.6 | 4.8 |
| | Polymer (C) | (C-1) | - | - | - | - | 3.8 | - | 3.7 | - |
| | | (C-2) | - | - | - | - | - | - | - | - |
| | | (C-3) | 3.2 | 3.2 | - | - | - | - | - | - |
| | Polyethylene (D) | | 4.8 | 4.8 | 5.0 | 5.0 | 5.8 | 6.0 | 5.5 | 5.7 |
| Spinning condition | Stretching air flow rate | [m/min] | 4,190 | 2,841 | 4,190 | 2,841 | 4,190 | 4,190 | 4,190 | 4,190 |
| | Spinnability | | A | A | B | A | A | A | A | A |
| Resin properties | (B) Number of islands | [count] | 289 | 301 | 266 | 283 | - | - | 234 | 102 |
| | (B) Average diameter of islands | [μm] | 0.124 | 0.133 | 0.126 | 0.129 | - | - | 0.088 | 0.125 |
| | (B) Area ratio of islands | [%] | 18.9 | 19.8 | 15.7 | 18.1 | - | - | 7.4 | 7.0 |
| | (D) Number of islands | [count] | 130 | 119 | 85 | 72 | 186 | 64 | 186 | 66 |
| | (D) Average diameter of islands | [μm] | 0.119 | 0.128 | 0.149 | 0.151 | 0.102 | 0.153 | 0.102 | 0.168 |
| | (110) plane peak intensity | [int[counts]] | 579 | 553 | 573 | 561 | 549 | 605 | 534 | 591 |
| | Crystallinity | [%] | 52 | 49 | 52 | 50 | 45 | 52 | 54 | 53 |
| | Peak intensity/crystallinity | [int[counts]/%] | 11.1 | 11.3 | 11.0 | 11.2 | 12.2 | 11.6 | 9.9 | 11.2 |
| | Fiber diameter | [μm] | | 30 | | 30 | 23 | 24 | 24 | 24 |

EP 4 484 627 A1

| | | | | Compara-tive Example 6 | Compara-tive Example 7 | Compara-tive Example 8 | Compara-tive Example 9 | Compara-tive Example 10 | Compara-tive Example 11 | Exam-ple 4 | Compara-tive Example 12 |
|---|---|---|---|---|---|---|---|---|---|---|---|
| Evalua-tion re-sults | Nonwo-ven fabric properties | Basis weight | [g/m²] | Non-woven fabric could not be pro-duced | 30 | Non-woven fabric could not be pro-duced | 30 | 30 | 30 | 29 | 30 |
| | | MD maximum strength | [N/25mm] | | 15 | | 16 | 27 | 28 | 29 | 28 |
| | | CD maximum strength | [N/25mm] | | 10 | | 10 | 10 | 10 | 11 | 11 |
| | | Strength index | [N/25mm] | | 13 | | 13 | 20 | 21 | 22 | 22 |
| | | Strength index/Basis weight | [N/25mm/g/-m²] | | 0.43 | | 0.44 | 0.68 | 0.68 | 0.73 | 0.72 |
| | | MD elongation | [%] | | 178 | | 189 | 217 | 224 | 239 | 211 |
| | | CD elongation | [%] | | 135 | | 141 | 166 | 165 | 165 | 153 |
| | | Air permeability | [cm³/cm²·s] | | 502 | | 489 | 262 | 258 | 304 | 269 |
| | | Uniformity index | [-] | | 425 | | 412 | 371 | 348 | 358 | 374 |
| | | Theoretical resin density | [g/cm³] | | 0.95 | | 0.95 | 0.91 | 0.91 | 0.92 | 0.92 |
| | | Thickness | [mm] | | 0.12 | | 0.12 | 0.12 | 0.12 | 0.13 | 0.11 |
| | | Filling rate | [%] | | 26 | | 26 | 27 | 28 | 24 | 26 |
| | | Porosity | [%] | | 74 | | 74 | 73 | 72 | 76 | 74 |

As shown in Tables 1 and 2, in the spun-bonded nonwoven fabrics of Examples 1 to 4, the resin composition contained the propylene polymer (A), the thermoplastic polyurethane-based elastomer (B), and the polymer (C). The ratio (peak intensity/crystallinity) was 10.5 (intensity(counts)/%) or lower. The stretching air flow rate was 3,000 m/min or more. Therefore, the ratio (strength index/basis weight) was 0.70 [(N/25 mm)/(g/m$^2$)] or higher in Examples 1 to 4. The air permeability was 280 cm$^3$/cm$^2$·sec or more in Examples 1 to 4. From these results, it was found that the spun-bonded nonwoven fabrics of Examples 1 to 4 had an excellent tensile strength and a high air permeability.

In the spun-bonded nonwoven fabric of Comparative Example 1, the resin composition contained the propylene polymer (A), the thermoplastic polyurethane-based elastomer (B), and the polymer (C). The ratio (peak intensity/crystallinity) was higher than 10.5 (intensity(counts)/%). The stretching air flow rate was less than 3,000 m/min. Therefore, in Comparative Example 1, the ratio (strength index/basis weight) was lower than 0.70 [(N/25 mm)/(g/m$^2$)].

In the spun-bonded nonwoven fabrics of Comparative Examples 2, 6, and 8, the resin composition contained the propylene polymer (A) and the thermoplastic polyurethane-based elastomer (B), but not the polymer (C). The ratio (peak intensity/crystallinity) was higher than 10.5 (intensity(counts)/%). The stretching air flow rate was 3,000 m/min or more. Therefore, in Comparative Examples 2, 6, and 8, a spun-bonded nonwoven fabric could not be produced due to the breakage of a large number of fibers in the spinning stage.

In the spun-bonded nonwoven fabrics of Comparative Examples 3, 7, and 9, the resin composition contained the propylene polymer (A) and the thermoplastic polyurethane-based elastomer (B), but not the polymer (C). The ratio (peak intensity/crystallinity) was higher than 10.5 (intensity(counts)/%). The stretching air flow rate was less than 3,000 m/min. Therefore, in Comparative Examples 3, 7, and 9, the ratio (strength index/basis weight) was lower than 0.70 [(N/25 mm)/(g/m$^2$)].

In the spun-bonded nonwoven fabrics of Comparative Examples 4 and 10, the resin composition contained the propylene polymer (A) and the polymer (C), but not the thermoplastic polyurethane-based elastomer (B). The ratio (peak intensity/crystallinity) was higher than 10.5 (intensity(counts)/%). The stretching air flow rate was 3,000 m/min or more. Therefore, the ratio (strength index/basis weight) was lower than 0.70 [(N/25 mm)/(g/m$^2$)] in Comparative Examples 4 and 10. The air permeability was less than 280 cm$^3$/cm$^2$·sec in Comparative Examples 4 and 10.

In the spun-bonded nonwoven fabric of Comparative Example 5, the resin composition contained the propylene polymer (A), but neither the thermoplastic polyurethane-based elastomer (B) nor the polymer (C). The ratio (peak intensity/crystallinity) was higher than 10.5 (intensity(counts)/%). The stretching air flow rate was 3,000 m/min or more. Therefore, in Comparative Example 5, the air permeability was less than 280 cm$^3$/cm$^2$·sec.

In the spun-bonded nonwoven fabric of Comparative Example 11, the resin composition contained the propylene polymer (A) and the polyethylene (D-1), but neither the thermoplastic polyurethane-based elastomer (B) nor the polymer (C). The ratio (peak intensity/crystallinity) was higher than 10.5 (intensity(counts)/%). The stretching air flow rate was 3,000 m/min or more. Therefore, the ratio (strength index/basis weight) was lower than 0.70 [(N/25 mm)/(g/m$^2$)] in Comparative Example 11. The air permeability was less than 280 cm$^3$/cm$^2$·sec in Comparative Example 11.

In the spun-bonded nonwoven fabric of Comparative Example 12, the resin composition contained the propylene polymer (A), the thermoplastic polyurethane-based elastomer (B), and the polyethylene (D-1), but not the polymer (C). The ratio (peak intensity/crystallinity) was higher than 10.5 (intensity(counts)/%). The stretching air flow rate was 3,000 m/min or more. Therefore, in Comparative Example 12, the air permeability was less than 280 cm$^3$/cm$^2$·sec.

From these results, it was found that the spun-bonded nonwoven fabrics of Comparative Examples 1 to 12 did not satisfy the feature of having an excellent tensile strength and a high air permeability.

The disclosure of Japanese Patent Application No. 2022-057478 filed on March 30, 2022 is hereby incorporated by reference in its entirety.

All the documents, patent applications, and technical standards that are described in the present specification are hereby incorporated by reference to the same extent as if each individual document, patent application, or technical standard is concretely and individually described to be incorporated by reference.

**Claims**

1. A spun-bonded nonwoven fabric, comprising fibers formed of a resin composition that comprises:

   a propylene polymer (A);
   a thermoplastic polyurethane-based elastomer (B); and
   a polymer (C) modified with at least one polar group selected from oxygen-containing groups, nitrogen-containing groups, sulfur-containing groups, or phosphorus-containing groups,
   wherein:

   a ratio of a peak intensity of $\alpha$-crystals of the fibers with respect to a crystallinity of the fibers is 10.5 (intensity(counts)/%) or lower, and

the peak intensity of the $\alpha$-crystals represents a peak intensity of a (110) plane of the propylene polymer (A) at approximately 2θ = 14° in an X-ray diffraction pattern measured by an X-ray diffractometer using CuK$\alpha$ radiation.

2. A spun-bonded nonwoven fabric, comprising fibers formed of a resin composition that comprises:

a propylene polymer (A);
a crystal nucleating agent (b); and
a polymer (C) modified with at least one polar group selected from oxygen-containing groups, nitrogen-containing groups, sulfur-containing groups, or phosphorus-containing groups,
wherein:

the fibers have a sea-island structure,
a sea phase contained in the sea-island structure comprises the propylene polymer (A),
island phases contained in the sea-island structure comprise the crystal nucleating agent (b),
a ratio of a peak intensity of $\alpha$-crystals of the fibers with respect to a crystallinity of the fibers is 10.5 (intensity(counts)/%) or lower, and
the peak intensity of the $\alpha$-crystals represents a peak intensity of a (110) plane of the propylene polymer (A) at approximately 2θ = 14° in an X-ray diffraction pattern measured by an X-ray diffractometer using CuK$\alpha$ radiation.

3. The spun-bonded nonwoven fabric according to claim 1, wherein:

a content of the propylene polymer (A) is from 70.0% by mass to 97.0% by mass with respect to a total amount of the resin composition,
a content of the thermoplastic polyurethane-based elastomer (B) is from 2.0% by mass to 20.0% by mass with respect to the total amount of the resin composition, and
a content of the polymer (C) is from 1.0% by mass to 10.0% by mass with respect to the total amount of the resin composition.

4. The spun-bonded nonwoven fabric according to claim 1 or 3, wherein, in a cross-section of a fiber perpendicular to the axial direction of the fiber observed under a transmission electron microscope in a viewing area of 25.5 $\mu$m$^2$;

island phases comprising the thermoplastic polyurethane-based elastomer (B) have an average diameter of 0.095 $\mu$m or less, and
a number of the island phases comprising the thermoplastic polyurethane-based elastomer (B) is 200 or more.

5. The spun-bonded nonwoven fabric according to any one of claims 1 to 4, wherein the polar group comprises at least one of oxygen-containing groups or nitrogen-containing groups.

6. The spun-bonded nonwoven fabric according to any one of claims 1 to 5, wherein the polar group comprises at least one of amino groups, carboxyl groups, carboxylic acid anhydride groups, or ester groups.

7. The spun-bonded nonwoven fabric according to claim 6, wherein the polar group comprises amino groups.

8. The spun-bonded nonwoven fabric according to any one of claims 1 to 7, wherein a main chain of the polymer (C) comprises a polyolefin or a hydrogenated styrene-based block copolymer.

9. The spun-bonded nonwoven fabric according to any one of claims 1 to 8, wherein the propylene polymer (A) comprises a propylene homopolymer having a melting point of 140°C or higher.

10. The spun-bonded nonwoven fabric according to any one of claims 1 to 9, wherein the resin composition further comprises a polyethylene (D) having a density of from 0.941 g/cm$^3$ to 0.970 g/cm$^3$.

11. The spun-bonded nonwoven fabric according to claim 10, wherein a content of the polyethylene (D) is from 1.0% by mass to 10.0% by mass with respect to a total amount of the resin composition.

12. The spun-bonded nonwoven fabric according to claim 10 or 11, wherein, in a cross-section of a fiber perpendicular to the axial direction of the fiber observed under a transmission electron microscope in a viewing area of 25.5 $\mu m^2$;

   island phases comprising the polyethylene (D) have an average diameter of 0.11 $\mu$m or less; and
   a number of the island phases comprising the polyethylene (D) is 150 or more.

13. The spun-bonded nonwoven fabric according to any one of claims 1 to 12, wherein the ratio of a peak intensity of $\alpha$-crystals of the fibers with respect to a crystallinity of the fibers is from 8.5 (intensity(counts)/%) to 10.5 (intensity(counts)/%).

14. A hygienic material, comprising the spun-bonded nonwoven fabric according to any one of claims 1 to 13.

15. A method of producing a spun-bonded nonwoven fabric, the method comprising:

   melt-spinning a resin composition to form a continuous fiber group; and
   stretching the continuous fiber group with cooling air,
   wherein:

   the resin composition comprises a propylene polymer (A), a thermoplastic polyurethane-based elastomer (B), and a polymer (C) modified with at least one polar group selected from oxygen-containing groups, nitrogen-containing groups, sulfur-containing groups, or phosphorus-containing groups, and
   the cooling air has a flow rate of 3,000 m/min or more.

# FIG.1

## INTERNATIONAL SEARCH REPORT

| International application No. |
|---|
| **PCT/JP2023/011623** |

**A.    CLASSIFICATION OF SUBJECT MATTER**

*D04H 3/16*(2006.01)i; *D01F 6/46*(2006.01)i; *D01F 8/06*(2006.01)i; *D01F 8/16*(2006.01)i; *D04H 3/007*(2012.01)i; *D04H 3/009*(2012.01)i; *D04H 3/147*(2012.01)i
FI:    D04H3/16; D01F6/46 D; D01F8/06; D01F8/16; D04H3/007; D04H3/009; D04H3/147

According to International Patent Classification (IPC) or to both national classification and IPC

**B.    FIELDS SEARCHED**

Minimum documentation searched (classification system followed by classification symbols)

D01F6/06;6/46;8/00-8/18;D04H1/00-18/04;C08K3/00-13/08;C08L1/00-101/14

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| A | JP 2012-501377 A (INTERFACE BIOLOGICS INC.) 19 January 2012 (2012-01-19) | 1-15 |
| A | JP 2004-244790 A (MITSUI CHEMICALS INC) 02 September 2004 (2004-09-02) | 1-15 |
| A | JP 2013-513676 A (BASF SE) 22 April 2013 (2013-04-22) | 1-15 |
| A | JP 53-8682 A (DUNLOP LTD) 26 January 1978 (1978-01-26) | 1-15 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **07 June 2023** | **20 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/011623**

| Patent document cited in search report | | | Publication date (day/month/year) | Patent family member(s) | | | Publication date (day/month/year) |
|---|---|---|---|---|---|---|---|
| JP | 2012-501377 | A | 19 January 2012 | US | 2011/0207893 | A1 | |
| | | | | WO | 2010/025398 | A1 | |
| | | | | EP | 2321360 | A1 | |
| | | | | CN | 102203153 | A | |
| JP | 2004-244790 | A | 02 September 2004 | US | 2006/0121812 | A1 | |
| | | | | WO | 2004/065679 | A1 | |
| | | | | EP | 1589140 | A1 | |
| | | | | KR | 10-2005-0088361 | A | |
| | | | | CN | 1742127 | A | |
| JP | 2013-513676 | A | 22 April 2013 | US | 2012/0283367 | A1 | |
| | | | | WO | 2011/069960 | A1 | |
| | | | | EP | 2509459 | A1 | |
| | | | | CN | 102651981 | A | |
| | | | | KR | 10-2012-0093429 | A | |
| JP | 53-8682 | A | 26 January 1978 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007091444 A
- DE 102019127827 A1
- JP 2004244791 A
- JP H08239414 A

- WO 2007114102 A
- WO 2003087172 A
- JP 2022057478 A

**Non-patent literature cited in the description**

- **A. ZAMBELLI et al.** *Macromolecules*, 1975, vol. 8, 687